# EUROPÄISCHE PATENTSCHRIFT

(11) **EP 1 232 158 B1**
(45) Veröffentlichungstag und Bekanntmachung des Hinweises auf die Patenterteilung: **25.02.2004**
(21) Anmeldenummer: 99960993.6
(22) Anmeldetag: 23.11.1999
(51) Int. Cl.: C07D 487/04, A61K 31/407, A61P 29/00

(54) **ANTIINFLAMMATORISCHE OXO- UND HYDROXYDERIVATE VON PYRROLIZINEN UND DEREN ANWENDUNG IN DER PHARMAZIE**
ANTI-INFLAMMATORY OXO DERIVATIVES AND HYDROXY DERIVATIVES OF PYRROLIZINES, AND THEIR PHARMACEUTICAL USE
DERIVES OXO ET HYDROXY ANTI-INFLAMMATOIRES DE PYRROLIZINES ET LEUR APPLICATION PHARMACEUTIQUE

(43) Veröffentlichungstag der Anmeldung: 21.08.2002
(60) Teilanmeldung: 03022605.4
(73) Patentinhaber: MERCKLE GMBH, D-89079 Ulm (DE)
(72) Erfinder: LAUFER, Stefan, D-89143 Blaubeuren (DE); NEHER, Karola, D-65611 Brechen (DE); STRIEGEL, Hans-Günter, D-89134 Blaustein (DE)
(74) Vertreter: Kinzebach, Werner, Dr.
(86) Internationale Anmeldenummer: PCT/EP1999/009057
(87) Internationale Veröffentlichungsnummer: WO 2001/005792

(56) Entgegenhaltungen:
- WO-A-95/32972
- DE-A- 19 845 446

## Beschreibung

Die vorliegende Erfindung betrifft antiinflammatorische Oxo- und Hydroxyderivate von Pyrrolizinen, pharmazeutische Mittel, welche diese Verbindungen enthalten und ihre Anwendung in der Pharmazie.

Pharmakologisch wirksame Pyrrolizinverbindungen, welche die 5-Lipoxygenase (5-LO) und die Cyclooxygenase-1 und -2 (Cox-1 und Cox-2) inhibieren, sind bereits bekannt. Beispielsweise werden antiphlogistisch wirksame Pyrrolizinverbindungen beschrieben in Arch. Pharm. 319, 231-234 (1986); 318, 661-663 (1985); 318, 663-664 (1985); 319, 500-505 (1986); 319, 749-755 (1986); 327, 509-514 (1994); 330, 307-312 (1997) sowie in J. Med. Chem. 1987, 30, 820-823 und 1994, 37, 1894-1897. Eine Verbindung dieses Typs ist die 6-(4-Chlorphenyl)-7-phenyl-2,3-dihydropyrrolo-[1,2-a]pyrrolverbindung ML 3000, s. Drugs of the Future, 1995, 20 (10): 1007-1009. Sie unterdrückt die Freisetzung von Leukotrienen, Thromboxanen und Prostaglandinen. Die inhibitorische Wirkung auf die Bildung der Leukotriene und der Prostaglandine ist bei dieser Struktur ausgewogen, schädliche Effekte einer reinen Hemmwirkung auf Cyclooxygenase-1 und -2 (Cox-1 bzw. Cox-2) mit vermehrter Bildung von Leukotrienen werden hier nicht beobachtet. Bei allen diesen Verbindungen ist die 1-Stellung des Pyrrolizingerüsces unsubstituiert.

Weitere Pyrrolizinverbindungen sind in der US 5,260,451 sowie in WO 95/32970; WO 95/32971; und WO 95/32972 beschrieben. Diese Verbindungen besitzen die Strukturformel

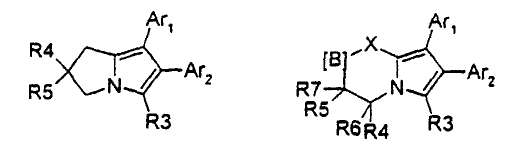

Allen Verbindungen ist ein anelliertes Diarylpyrrol-Strukturelement und ein dritter acider Rest R3 gemeinsam. Die Verbindungen zeichnen sich durch hohe Lipophilie, gute Bioverfügbarkeit und mittlere Halbwertzeiten aus.

Gemäß der allgemeinen Offenbarung in WO 95/32970 und WO 95/32971 kann X eine Carbonylgruppe bedeuten. Verbindungen dieses Typs sind jedoch nach den in den oben genannten WO-Publikationen und in der US 5,260,451 beschriebenen Verfahren nicht herstellbar.

Die US 3,705,905 offenbart Verbindungen der Formel 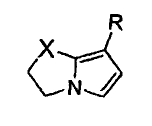 worin X für Methylen, α- oder β-Hydroxyethylen oder Carbonyl steht und R eine Hydroxymethylgruppe oder eine Formylgruppe bedeutet. Diese Verbindungen besitzen Antivirus-, Antitumor- oder immunosuppressive Aktivität.

Weitere Pyrrolizinverbindungen, die zur Behandlung thrombotischer Erkrankungen brauchbar sind, sind in US 4,546,100 und US 4,684,658 offenbart.

Aufgabe der vorliegenden Erfindung war es, Verbindungen zur Verfügung zu stellen, die im Vergleich zum Stand der Technik eine ebenso ausgewogene Wirkung auf hohem Niveau auf die beiden Schlüssel-Enzymsysteme der Arachidonsäurekaskade, nämlich die 5-Lipoxygenase und die Cyclooxygenasen ausüben.

Überraschenderweise wurde nun gefunden, dass diese Aufgabe durch Pyrrolizinverbindungen gelöst wird, welche in verschiedenen Positionen des Ringskelettes Sauerstofffunktionen als polaritätserhöhende Gruppen, wie eine Carbonylgruppe, eine Hydroxygruppe oder eine Alkoxygruppe, aufweisen.

Gegenstand der vorliegenden Erfindung sind daher Pyrrolizinverbindungen der Formel I 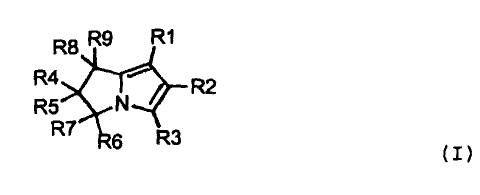 worin
- R1 und R2,: die gleich oder verschieden sein können, für Aryl oder einen aromatischen mono- oder bicyclischen, heterocyclischen Rest stehen, der 1, 2 oder 3 Heteroatome aufweist, die unabhängig voneinander ausgewählt sind unter N, O und S, wobei die Reste R¹ und R2 gegebenenfalls durch 1, 2 oder 3 Gruppen, die unabhängig voneinander ausgewählt sind unter Alkyl, Halogen, CF₃, Hydroxy, Alkoxy, Aryloxy und CN, substituiert und gegebenenfalls mit Phenyl oder Naphthyl kondensiert sein können;
- R3: für H, Alkyl, COOH, COOAlkyl, COOAlkPh, COCOOAlkyl, CHO oder A-Y steht, wobei
A für C₁-C₉-Alkylen oder C₂-C₈-Alkenylen steht, das gegebenenfalls durch Hydroxyl oder Alkoxy substituiert sein kann,
Y für COOH, SO₃H, OPO(OH)₂, OP(OH)₂, Tetrazolyl, COOAlkyl, SO₃Alkyl, CHO oder OH steht;
Alk für C₁-C₄-Alkylen steht;
- R4, R5, R6 und R7,: die gleich oder verschiedenen sein können, für H, Alkyl, Hydroxyalkyl oder Alkoxyalkyl stehen;
einer der Reste R8 und R9 für H, Alkyl, Hydroxyalkyl oder Alkoxyalkyl steht und der andere für Hydroxyl, Alkoxy, Carboxyl oder Acyloxy steht oder R8 und R9 zusammen mit dem Kohlenstoffatom, an das sie gebunden sind, für eine Carbonylgruppe stehen,
und die optischen Isomere, physiologisch verträglichen Salze und physiologisch leicht hydrolysierbaren Ester davon.

Die physiologisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Milchsäure, Citronensäure, Äpfelsäure, Mandelsäure, Ascorbinsäure, Maleinsäure, Fumarsäure, Gluconsäure und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Physiologisch leicht hydrolysierbare Ester der Verbindungen der Formel I sind beispielsweise Alkyl-, Pivaloyloxymethyl-, Acetoxymethyl-, Phthalidyl-, Indanyl- und Methoxymethylester.

Soweit die erfindungsgemäßen Verbindungen Asymmetriezentren aufweisen, sind Racemate sowie optische Isomere (Enantiomere, Diastereomere) umfasst.

Der Ausdruck "Alkyl, Alkoxy etc." umfasst geradkettige oder verzweigte Alkylgruppen, wie Methyl, Ethyl, n- und i-Propyl, n-, i-oder t-Butyl, n-Pentyl, Neopentyl, n-Hexyl etc.

Soweit nicht anders angegeben, steht "Alkyl" vorzugsweise für C₁-C₈-Alkyl, insbesondere C₁-C₆-Alkyl und besonders bevorzugt für C₁-C₄-Alkyl.

"Aryl" steht vorzugsweise für Naphthyl und insbesondere für Phenyl.

Der Ausdruck "Halogen" umfasst ein Fluor-, Chlor-, Brom- oder Iodatom und insbesondere ein Fluor- oder Chloratom.

"Alkylen" oder "Alkenylen" steht für geradkettige oder verzweigte Alkylen- oder Alkenylengruppen mit vorzugsweise 1 bis 6 bzw. 2 bis 6 und insbesondere 1 bis 4 bzw. 2 bis 4 Kohlenstoffatomen. Die Alkylengruppe und insbesondere die Methylengruppe ist bevorzugt.

"Acyl" steht für RCO, wobei R vorzugsweise die oben für "Alkyl" und "Aryl" angegebenen Bedeutungen besitzt. Acetyl ist besonders bevorzugt.

Bei dem "aromatischen heterocyclischen Rest" handelt es sich insbesondere um einen 5- und 6-gliedrigen heterocyclischen Rest, der wie oben angegeben substituiert und kondensiert sein kann. Beispiele sind ein Thiophen-, Pyrrol-, Imidazol-, Thiazol-, Thiadiazol, Furan-, Oxazol-, Isoxazol-, Pyridin-, Pyrimidin-, Benzofuran- oder Chinolinrest. Wenn der Heterozyklus substituiert ist, sind 1, 2 oder 3 Substituenten vorhanden, die ausgewählt sind unter Halogen, CF₃, C₁-C₈-Alkyl und C₁-C₉-Alkoxy. Bevorzugt ist ein Thiophen- oder Halogen-, insbesondere chlorsubstituierter, Thiophenrest, ein Furan-, Pyridin-, Benzofuran- oder Chinolinrest.

Die Substituenten der Arylgruppe sind vorzugsweise ausgewählt unter Halogen, insbesondere Fluor oder Chlor und CF₃. Wenn es sich bei der Arylgruppe um einen Phenylring handelt, befinden sich die Substituenten vorzugsweise in m- und/oder p-Stellung.

Besonders bevorzugt stehen R1 und R2 für Phenyl, halogensubstituiertes Phenyl (substituiert mit 1 oder 2 Halogenatomen, insbesondere Chloratomen), hydroxy- oder alkoxysubstituiertes Phenyl (substituiert mit 1 oder 2 Hydroxy- oder Alkoxygruppen), Thienyl oder Benzofuranyl. Insbesondere bevorzugt stehen R1 für Phenyl und R2 für Phenyl, das mit 1 oder 2 Halogenatomen, insbesondere Chloratomen, und/oder 1 oder 2 Hydroxygruppen substituiert ist.

Verbindungen der Formel I, worin R3 für COOAlkyl, COOAlkPhenyl, COCOOAlkyl oder A-Y, wobei A und Alk die oben angegebenen Bedeutungen besitzen und Y für COOAlkyl, SO₃Alkyl oder OH steht, sind Zwischenprodukte, die für die Herstellung der pharmakologisch wirksamen Verbindungen der Formel I brauchbar sind. Pharmakologisch wirksame Verbindungen der Formel I sind somit solche, worin R3 für H, Alkyl, COOH, CHO oder A-Y steht, wobei A die oben angegebenen Bedeutungen besitzt und Y für COOH, SO₃H, OPO(OH)₂, PO(OH)₂, CHO oder Tetrazolyl steht.

Vorzugsweise steht R3 für H, Alkyl, COOH, CHO oder A-Y, wobei A für C₁-C₈-Alkylen steht, das gegebenenfalls durch OH substituiert ist, und Y für COOH steht.

Die Reste R6 und R7 stehen vorzugsweise für H und die Reste R4 und R5 vorzugsweise für Alkyl, insbesondere Methyl.

Eine bevorzugte Ausführungsform sind Verbindungen der Formel I, worin R1 für Phenyl, R2 für Phenyl, das mit 1 oder 2 Halogenatomen, insbesondere Chloratomen, und/oder 1 oder 2 Hydroxy- und/oder Alkoxygruppen substituiert ist; R3 für H, COOH, CHO, CH₂COOH oder CH(OH)COOH steht; und R4 bis R9 die oben angegebenen Bedeutungen besitzen.

Eine weitere Ausführungsform sind Verbindungen der Formel I, worin wenigstens einer der Reste R4, R5, R6 und R7 für Hydroxyalkyl, insbesondere Hydroxymethyl steht, und die anderen der Reste R4, R5, R6 und R7 unabhängig voneinander für H oder Alkyl stehen. Vorzugsweise steht R4 für Hydroxyalkyl, insbesondere Hydroxymethyl, R5 für H oder Alkyl und R6 und R7 unabhängig voneinander für H oder Alkyl. R1, R2 und R3 besitzen bevorzugt die im voranstehenden Absatz angegebenen Bedeutungen.

Schema A zeigt verschiedene Strategien zur Einführung solcher polaritätserhöhender funktioneller Gruppen in das Skelett des Pyrrolizingrundkörpers und seiner Derivate (A - F).

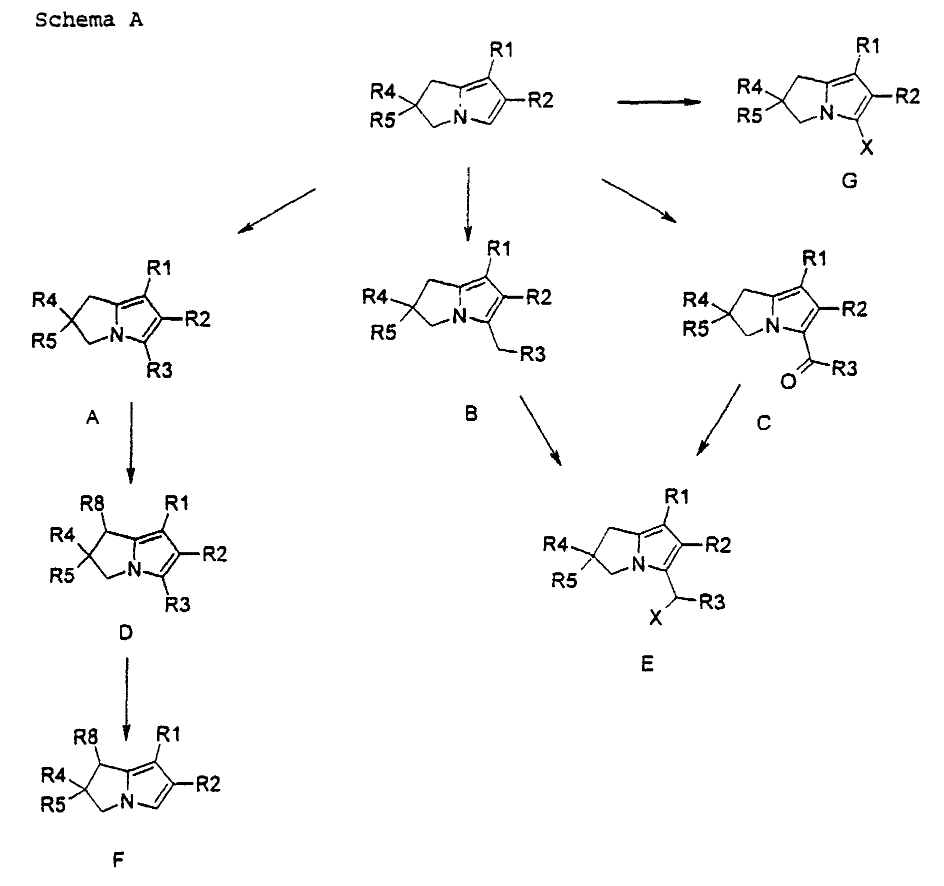

Die Herstellung der erfindungsgemäßen Verbindungen kann durch Oxidation in 1-Position erfolgen. Als Ausgangsverbindungen dienen dabei Verbindungen der Formel II, siehe das nachfolgende Syntheseschema 1. Die Herstellung der Verbindungen der Formel II erfolgt gemäß den im oben genannten Stand der Technik und insbesondere gemäß den in US 5,260,451 und WO 95/32970, WO 95/32971 und WO 95/32972 beschriebenen verfahren.

Zur Herstellung von Verbindungen der Formel I, worin R3 für COOH, COOAlkyl, COOAlkPhenyl, COCOOAlkyl oder CHO steht, werden die Verbindungen der Formel II in bekannter Weise mit N-Bromsuccinimid (NBS) in Gegenwart eines Radikalstarters, wie Azo-bis-isobutyronitril in einem chlorierten Lösungsmittel, wie Tetrachlorkohlenstoff, umgesetzt. Die Reaktionstemperatur wird so gewählt, dass die radikalische Reaktion abläuft, beispielsweise bei 70 bis 90 °C.

Bei Verwendung von 1 Moläquivalent N-Bromsuccinimid erhält man die in 1-Position bromierte Verbindung der Formel III. Diese wiederum kann dann mit Acylaten, beispielsweise Natriumacetat oder Alkoholacen, wie Natriummethylat oder Natriumethylat, zu den entsprechenden Verbindungen der Formel Ic und Id umgesetzt werden. Die Umsetzungen werden in bekannter Weise durchgeführt, die Umsetzung mit den Acylaten erfolgt in einem inerten Lösungsmittel, beispielsweise Dimethylformamid (DMF), bei 70 bis 90 °C. Die Umsetzung der Verbindungen der Formel III mit Alkoholaten erfolgt zweckmäßigerweise in dem entsprechenden Alkohol. Alternativ können die Verbindungen der Formel III, ohne aus der Reaktionsmischung isoliert zu werden, direkt mit einem Alkohol zu den Verbindungen der Formel Id umgesetzt werden.

Alternativ kann man eine Verbindung der Formel II mit einem Moläquivalent NBS in einem chlorierten Lösungsmittel in Anwesenheit von Wasser zu einer Verbindung der Formel Ia umsetzen. Mit einem weiteren Moläquivalent NBS erhält man dann eine Verbindung der Formel Ib.

In den Syntheseschemata hat Acyl die oben angegebenen Bedeutungen. R steht für einen Alkylrest.

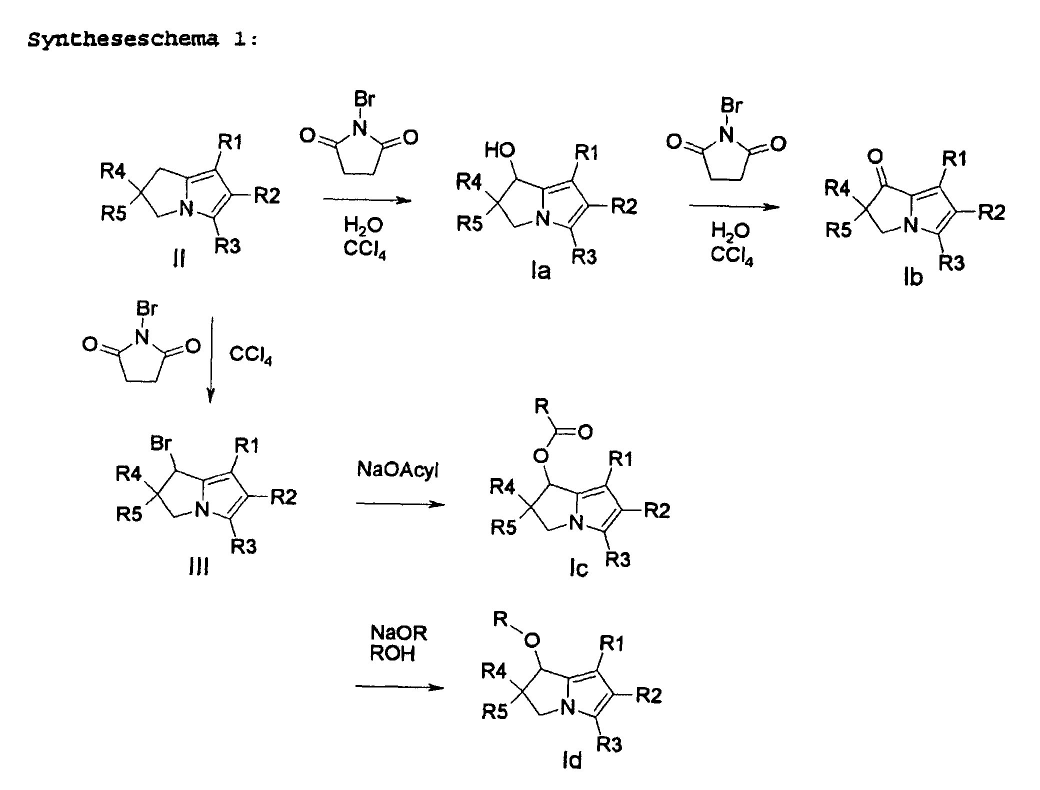

Erfindungsgemäße Verbindungen der Formel I, worin R3 für ein Wasserstoffatom steht, werden durch Oxidationsmittel bevorzugt am Pyrrolkern angegriffen, siehe Arch. Pharm. 318, 661-663 (1985); 663-664 (1985); 319, 231-234 (1986) und 312, 896-907 (1985). Verbindungen der Formel I, worin R3 für einen aliphatischen Rest (beispielsweise CH₃COOH, CH₃) steht, werden bevorzugt an diesen aliphatischen Substituenten angegriffen. Zur Herstellung von Verbindungen der Formel I, worin R3 für ein Wasserstoffatom steht, wird daher die reaktive 5-Position zunächst blockiert. Wie im Syntheseschema 2 gezeigt, erfolgt dies ausgehend von Verbindungen der Formel V, deren Herstellung ebenfalls in dem oben genannten Stand der Technik und insbesondere in US 5,269,451 und WO 95/32970, WO 95/32971 und WO 95/32972 beschrieben ist, durch Einführung einer Acylfunktion. Zu diesem Zweck wird eine Verbindung der Formel V mit Chlorameisensäuretrichlormethylester in einem inerten Lösungsmittel, beispielsweise Tetrahydrofuran oder Dioxan, umgesetzt und der intermediär erhaltene Ester mit Methanol zur Reaktion gebracht, wobei man eine Verbindung der Formel VI erhält. Diese wird dann analog Syntheseschema 1 mit 2 Moläquivalenten N-Bromsuccinimid in Tetrachlorkohlenstoff und in Anwesenheit von Wasser zu einer Verbindung der Formel Ie umgesetzt. Durch basische Esterspaltung in üblicher Weise, beispielsweise mit Kaliumhydroxid in methanol oder Wasser, erhält man die Carbonsäure der Formel If. Diese wiederum kann bei 250 bis 350 °C zu einer Verbindung der Formel Ig (R3 = H) decarboxyliert werden. Die Verbindungen der Formel Ig können dann mit üblichen Methoden an der Position 5 funktionalisiert werden, beispielsweise durch Vilsmeyer-Formylierung mit Phosphoroxichlorid in Dimethylformamid (DMF) zu einer Verbindung der Formel Ix. Alternativ kann die Ketogruppe in 1-Position der Verbindung Ig zur Alkoholfunktion reduziert werden, beispielsweise mit komplexen Hydriden, wie Lithiumaluminiumhydrid. Die Alkoholfunktion in 5-Position kann dann in üblicher Weise verethert oder verestert werden, wobei man Verbindungen der Formel Ic und Id (R3 = H) erhält.

Alternativ kann die Verbindung der Formel VI mit einem Moläquivalent N-Bromsuccinimid und anschließend mit einem Alkoholat zu einer Verbindung der Formel Ii umgesetzt werden. Durch Esterspaltung und Decarboxylierung in der oben angegebenen Weise erhält man die Verbindungen der Formel Ij und Ik.

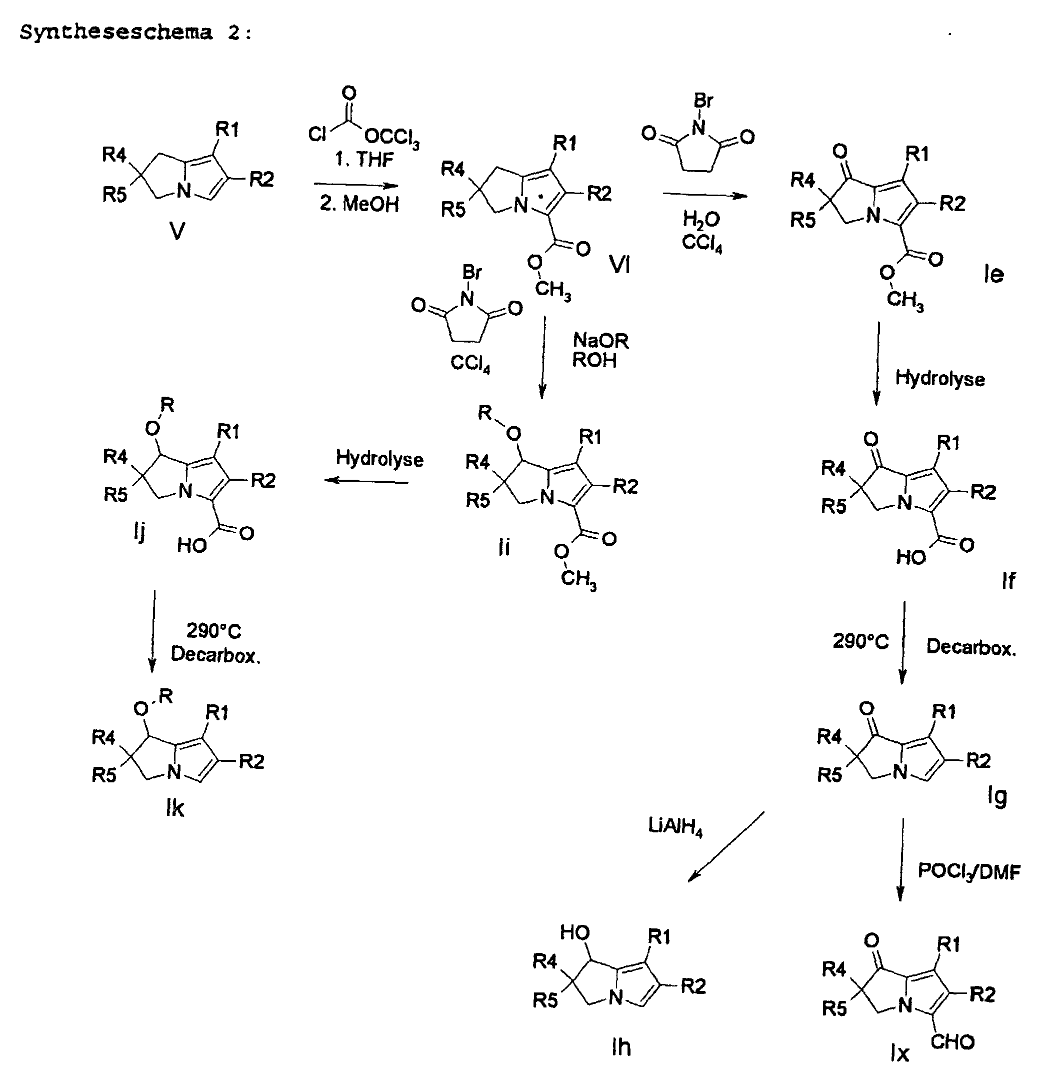

Eine Alternative zur Einführung einer Acylfunktion in eine Verbindung der Formel V besteht in dem im Syntheseschema 3 gezeigten Weg. Dabei wird eine Verbindung der Formel V mit Oxalsäureethylesterchlorid in einem inerten Lösungsmittel, wie THF oder Dioxan, zu einer Verbindung der Formel XV umgesetzt. Diese wiederum wird, wie bereits im Zusammenhang mit dem Syntheseschema 2 beschrieben, mit 2 Moläquivalenten N-Bromsuccinimid zu einer Verbindung der Formel Il oxidiert, die nach Hydrolyse (basische Esterspaltung wie oben beschrieben) eine Verbindung der Formel Im ergibt. Alternativ kann eine Verbindung der Formel XV mit einem Moläquivalent N-Bromsuccinimid und anschließend mit einem Alkoholat zu einer Verbindung der Formel In umgesetzt werden, die in üblicher Weise zu der freien Säure Io hydrolysiert werden kann. Die Verbindung der Formel Io kann weiter derivatisiert werden durch Reduktion der α-Ketogruppe des Substituenten in 5-Position durch Huang Minlon-Reduktion mit Hydrazin und Base. Man erhält dabei eine Verbindung der Formel Ip. Eine Reduktion einer Verbindung der Formel Io mit einem komplexen Hydrid, beispielsweise Natriumborhydrid in Wasser oder einem wässrigen Alkohol, ergibt die verbindung Iq.

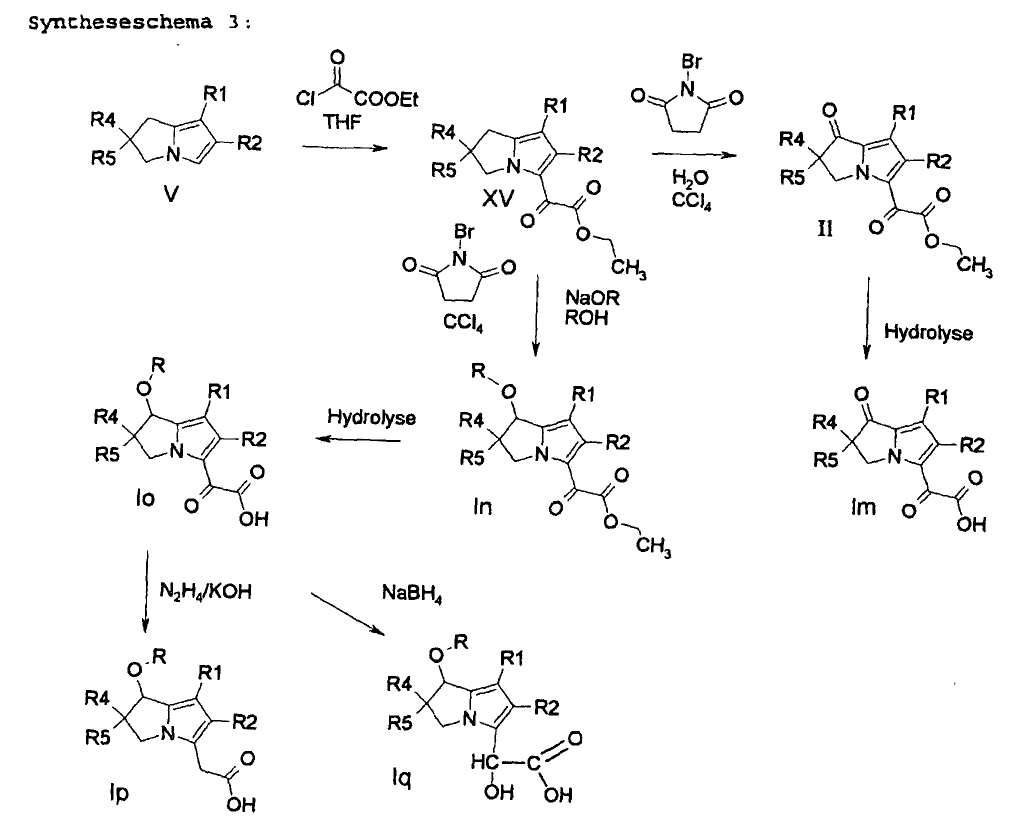

Der oben bereits erwähnte oxidative Angriff von N-Bromsuccinimid auf aliphatische Substituenten in 5-Position der erfindungsgemäßen Verbindungen lässt sich durch Einführung stark raumerfüllender Schutzgruppen im Bereich dieser Position verhindern. Die Synthese erfolgt dabei ausgehend von verbindungen der Formel IV, deren Herstellung ebenfalls im oben genannten Stand der Technik und insbesondere in US 5,260,451 und WO 95/32970, WO 95/32971 und WO 95/32972 beschrieben ist, nach den im Syntheseschema 4 beschriebenen Verfahren. Dabei wird eine Verbindung der Formel IV mit einem stark verzweigten Alkohol, wie t-Butanol oder Neopentylalkohol, in Anwesenheit eines Aktivierungsmittels, beispielsweise Carbonyldiimidazol, in einem inerten Lösungsmittel, wie Tetrahydrofuran oder Dioxan, zu einer Verbindung der Formel XXI verestert. Der Ester XXI wird, wie im Zusammenhang mit Syntheseschema 2 beschrieben, mit 2 Moläquivalenten N-Bromsuccinimid in eine Verbindung der Formel Ir überführt, die nach Esterspaltung und Decarboxylierung eine verbindung der Formel Is bzw. It ergibt. Alternativ kann der Ester XXI mit einem Moläquivalent N-Bromsuccinimid und anschließend mit Alkoholat zu einer verbindung der Formel Iu umgesetzt werden. Esterhydrolyse und Decarboxylierung ergibt dann eine Verbindung der Formel Iv und Iw.

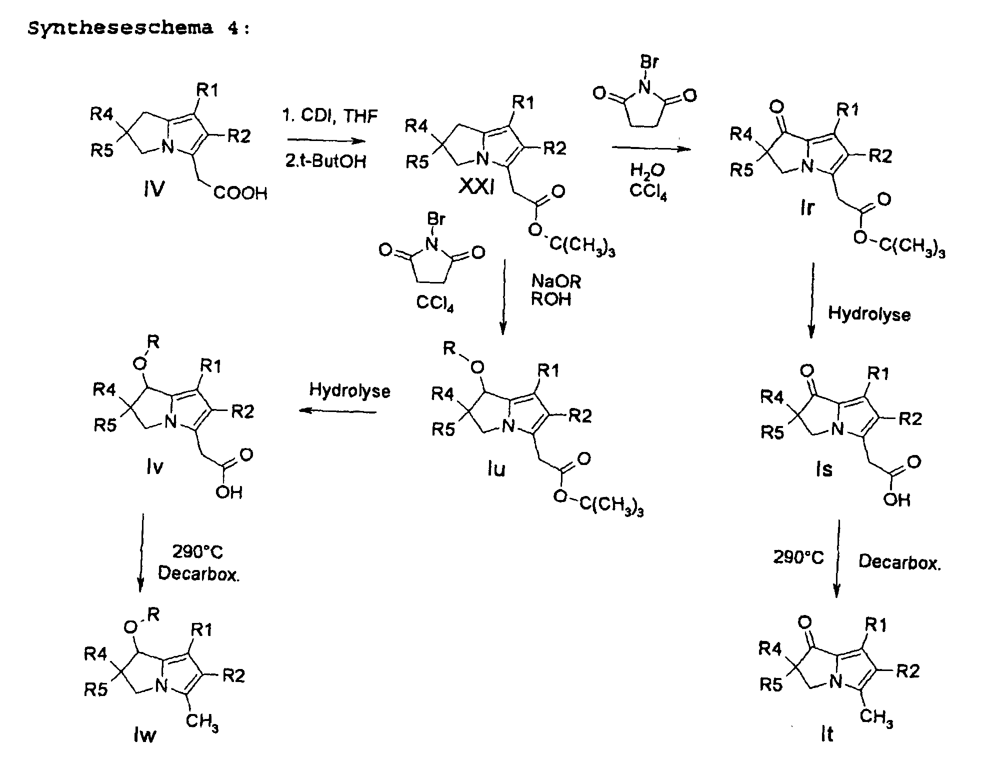

Die Einführung von polaren Gruppen, wie X bzw. R8 = Hydroxyl (Schema A), R8 und R9 = Carbonyl und R8 = Carboxyl setzt eine abgewandelte Synthesestrategie voraus. Solche funktionellen Gruppen lassen sich am Kohlenwasserstoff-Gerüst des Annelanden nicht nachträglich einführen. Die Herstellung gelingt über geeignete Vorstufen, die diese funktionellen Gruppen, geschützt durch sog. Schutzgruppen (=Pg *protective groups*), bereits enthalten.

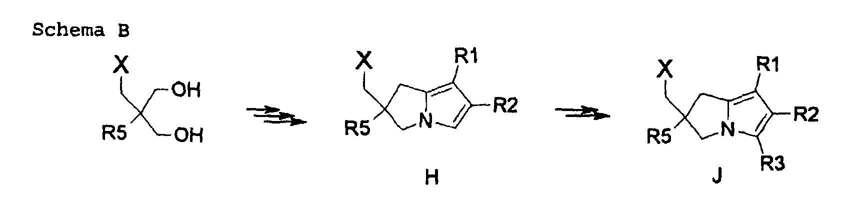

Syntheseschema 5 zeigt ein Beispiel, wie, in Anwendung der in EP 0397175 beschriebenen Methodik (XXXVII - XXXVIII, ausgehend von Tris-Hydroxymethylen-Derivaten (XXXIII, X=OH) durch selektive Einführung einer Schutzgruppe für eine der 3 Hydroxymethylengruppen, Verbindungen der Struktur H und J (Schema B) erhalten werden können, die das entsprechende Substitutionsmuster zeigen. Verbindungen der Struktur H und J treten ebenfalls in Form zweier optischer Antipoden auf, die bei Bedarf durch Anwendung chromatographischer Techniken getrennt werden oder über den Einsatz diastereomerer syntheseintermediate (siehe Syntheseschema 5, Formeln XXXV, XXXVI, Acetale oder Ketale mit R=H, R'=Alkyl, Aryl, oder R≠R'=Alkyl, Aryl etc.) enantiomerenrein synthetisiert werden.

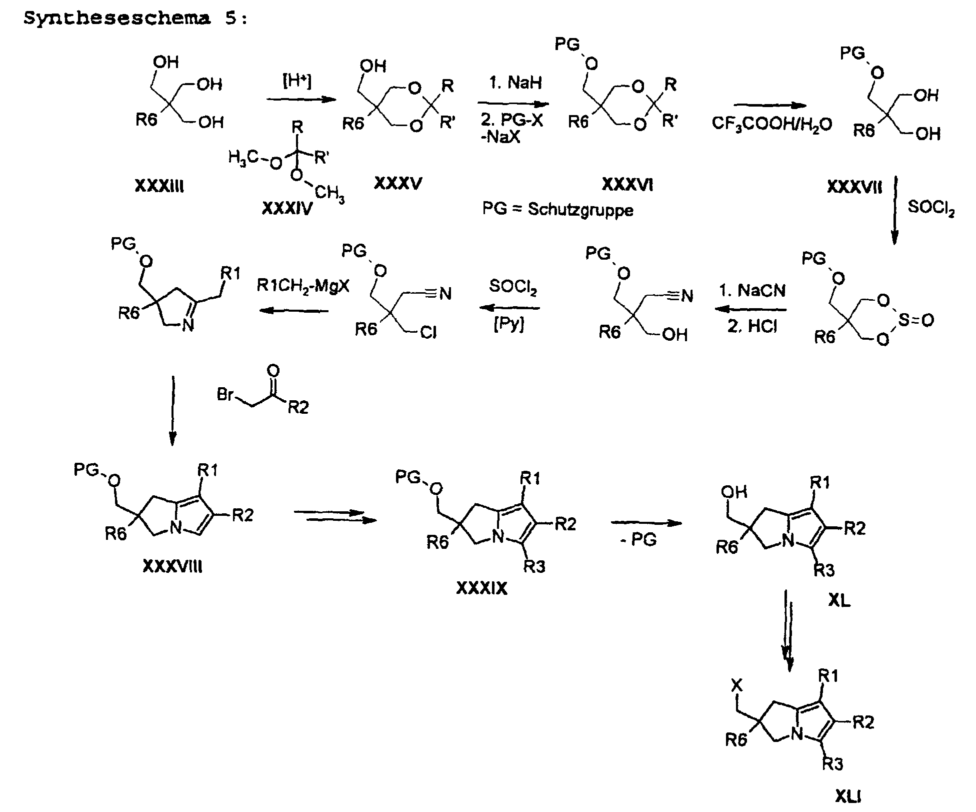

Die Einführung einer Hydroxygruppe (Schema A: Struktur G (X=OH) in den Pyrrolteil des Pyrrolizingrundgerüstes liefert Verbindungen (XXIX in Syntheseschema 6), die im Gleichgewicht zu tautomeren Oxoverbindungen stehen. Im Falle der Pyrrolizine können diese Verbindungen nur in Form stabiler Derivate isoliert werden. Sie erfahren eine rasche Umwandlung durch Oxidation mit Luftsauerstoff. Syntheseschema 6 beschreibt, wie an Pyrrolizinen mit Struktur v, die metalliert wurden, durch Einwirkung organischer Peroxyde direkt stabile Ester der entsprechenden Hydroxyderivate erhalten werden können.

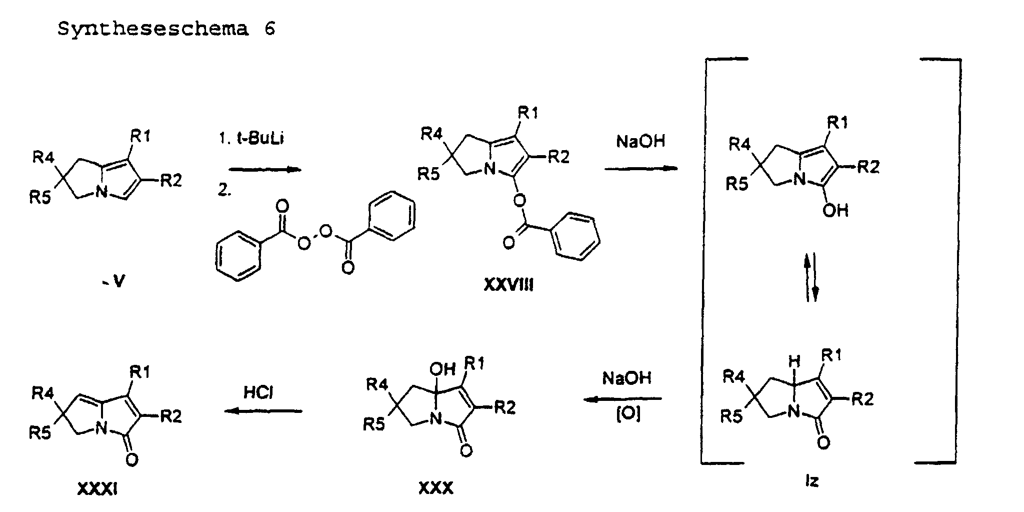

Syntheseschema 7 beschreibt, dass auch Substituenten in Position 5 (wie die Essigsäureseitenkette R³ = CH₂COOH, IIa) durch Behandlung mit Carbonsäuren in Anwesenheit von BF₃-Etherat gegen die Acylreste in der Weise getauscht werden, dass die Acyloxyderivate der zugrundeliegenden Pyrrolizine (Struktur XXXII) gebildet werden.

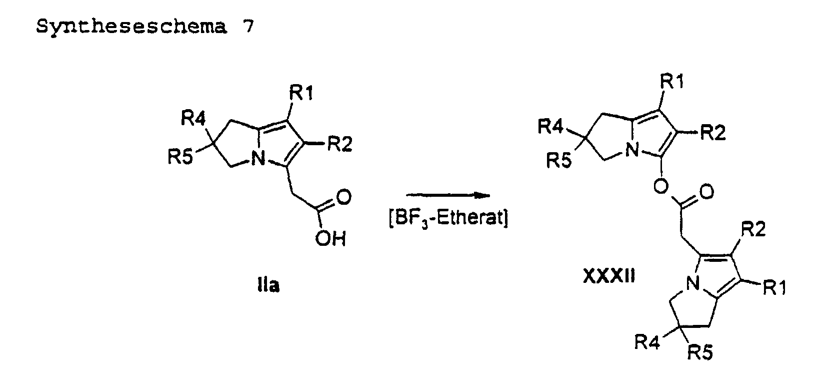

Die erfindungsgemäßen Verbindungen zeigen in vitro und in vivo Hemmung der Freisetzung verschiedener Mediatoren der Arachidonsäurekaskade, insbesondere der 5-Lipoxygenase und der Cyclooxygenase-1 und -2 und daher eine ausgeprägte antiinflammatorische Aktivität. Sie eignen sich somit zur Behandlung von Erkrankungen, bei denen erhöhte Freisetzungsraten der Eicosanoid-Mediatoren für die Entstehung bzw. den progredienten Verlauf dieser Erkrankungen verantwortlich sind. Insbesondere sind die erfindungsgemäßen Verbindungen zur Behandlung von Erkrankungen des rheumatischen Formenkreises und zur Prävention von allergisch induzierten Erkrankungen brauchbar. Die erfindungsgemäßen Verbindungen stellen somit wirksame Antiphlogistika, Analgetika, Antipyretika, Antiallergika und Broncholytika dar und daher zur Prophylaxe des anaphylaktischen und septischen Schocks sowie zur Behandlung dermatologischer Erkrankungen, wie Psoriasis, Urtikaria, akute und chronische Exantheme allergischer und nicht-allergischer Genese brauchbar.

Die erfindungsgemäßen Verbindungen besitzen erhöhte chemische Stabilität, parenterale Anwendbarkeit, verbesserte enterale Bioverfügbarkeit und kürzere Halbwertszeiten.

Die erfindungsgemäßen Verbindungen können entweder als einzelne therapeutische Wirkstoffe oder als Mischungen mit anderen therapeutischen Wirkstoffen verabreicht werden. Sie können als solche verabreicht werden, im Allgemeinen werden sie jedoch in Form pharmazeutischer Mittel verabreicht, d. h. als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z. B. Sirup, Akazia, Gelatine, Sorbit, Tragant oder Polyvinylpyrrolidon), Füllstoffe (z. B. Lactose, Zucker, Maisstärke, Calciumphosphat, Sorbit oder Glycin), Gleitmittel (z. B. Magnesiumstearat, Talcum, Polyethylenglykol oder Siliciumdioxid) , disintegrierende Mittel (z. B. Stärke) oder Netzmittel (z. B. Natriumlaurylsulfat). Orale flüssige Präparate können in Form wässriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Das Wirkungsspektrum der Verbindungen wurde anhand folgender Testsysteme untersucht:

### Testsystem zur Bestimmung der Hemmung der 5-Lipoxygenase

Als Quelle für die 5-Lipoxygenase dienen menschliche Granulozyten. Durch Stimulation mit Calcium-Ionophor A 23187 wird LTB₄ (Leukotrien B₄) aus endogener Arachidonsäure gebildet. Die Isolierung der Granulozyten und die Durchführung der Enzymreaktion erfolgt nach bekannten Verfahren (siehe Arch. Pharm. Med. Chem. 330, 307-312 (1997)).

Das mit Heparin vor Gerinnung geschützte Blut wird über einem diskontinuierlichen Percoll®-Gradienten zentrifugiert und die Granulozytenschicht abpipettiert. Nach Lyse der Erythrozyten werden die Granulozyten mehrmals gewaschen und anschließend auf eine bestimmte Zellzahl eingestellt. Die Enzymreaktion wird dann in An- bzw. Abwesenheit der Testsubstanz nach Zugabe von Ca²⁺ mit Calcium-Ionophor A 23187 gestartet. Die Synthese der Leukotriene wird nach 1,5 Minuten gestoppt. Die Proben werden abzentrifugiert, der Überstand verdünnt. Die quantitative Bestimmung von LTB₄ erfolgt mittels ELISA.

### Testsystem zur Bestimmung der Hemmung der Cyclooxygenase-1

Bei diesem Testsystem wird die von menschlichen Thrombozyten nach Zusatz von Calcium-Ionophor gebildete Prostaglandin E₂-Menge mittels ELISA bestimmt. Dabei werden die Thrombozyten nach Zentrifugation über einen diskontinuierlichen Percoll®-Gradienten gewonnen. Die Enzymreaktion und die Bestimmung der gebildeten Metaboliten erfolgt prinzipiell wie bei der Bestimmung der 5-Lipoxygenase-Hemmung. Unterschiede bestehen hinsichtlich der Inkubationszeit. Weiterhin ist die Zugabe eines Thromboxansynthase-Hemmstoffes notwendig (siehe Arch. Pharm. Med. Chem. 330, 307-312 (1997)).

### Testsystem zur Bestimmung der Hemmung der Cyclooxygenase-2

COX₂ (aus Placenta des Schafs) wird mit Testsubstanz 10 min bei 4 °C vorinkubiert, dann mit Arachidonsäure (5 µM) bei 25 °C 10 min stimuliert. Als Referenz dient Diclofenac (IC₅₀(COX₂) = 3,0 10⁻⁶ M). Die Bestimmung erfolgt in 3 Verdünnungen (10⁻⁷, 10⁻⁶, 10⁻⁵ M), Die PGE₂-Konzentrationen werden mittels ELISA quantifiziert (siehe Mitchell, J.A. et al. Proc. Nat. Acad. Sci 90: 11693-11697 (1993)).

### Bestimmung des Verteilungskoeffizienten

Der Verteilungskoeffizient P der Verbindungen wurde im System n-Octanol/Wasser nach der OECD-Guidline for Testing Chemicals, Nr. 117, bestimmt.

Die Ergebnisse sind in der nachfolgenden Tabelle 1 zusammengestellt.

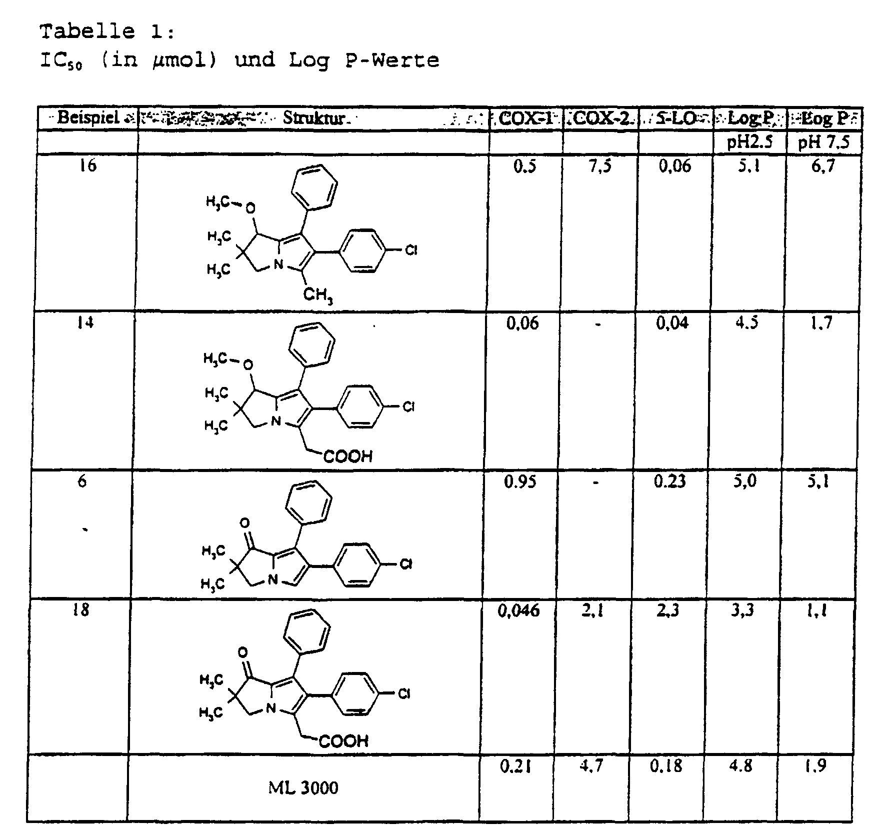

Die nachfolgenden Beispiele erläutern die Erfindung, ohne sie zu begrenzen.

In den Beispielen werden folgende Abkürzungen verwendet:
- THF: Tetrahydrofuran
- MeOH: Methanol
- NBS: N-Bromsuccinimid
- DMSO: Dimethylsulfoxid
- DMF: Dimethylformamid
- AIBN: Azobisisobutyronitril
- Smp: Schmelzpunkt

### Beispiel 1:

### 6-(4-Chlorphenyl)-2,3-dihydro-1-methoxy-2,2-dimethyl-7-phenyl-1H-pyrrolizin-5-yl]-carbonsäuremethylester

### a) 6-(4-Chlorphenyl)-2,3-dihydro-2,2-dimethyl-7-phenyl-1H-pyrrolizin-5-yl]-carbonsäuremethylester

(6-(4-Chlorphenyl)-2,3-dihydro-2,2-dimethyl-7-phenyl-1*H*-pyrrolizin (10.0 g, 31.1 mmol, hergestellt nach Laufer et al. ( J. Med. Chem. 1994, 37, 1894-1897) und 3.3 g trockenes Triethylamin werden in 30 mL absolutem THF gelöst. Dann tropft man bei Raumtemperatur langsam eine Lösung von 3.1 g (15.7 mmol) Chlorameisensäuretrichlormethylester (Diphosgen) in 20 mL THF zu und rührt 7 h nach. Anschließend werden 30 mL MeOH zugesetzt. Man rührt nochmals 12 h bei Raumtemperatur. Das Produkt fällt als hellgrüner Feststoff aus. Es wird vom Lösungsmittel abgesaugt und mit wenig MeOH gewaschen. Die mit 200 mL Wasser versetzte Mutterlauge liefert nach Ultraschallbehandlung (10 min.) eine zweite Kristallfraktion. Die so erhaltenen Fraktionen werden durch Ausrühren mit MeOH gereinigt.

Gesamtausbeute: 9.94 g (84 %), C₂₃H₂₂ClNO₂, 379.9 g/mol,
Smp.: 170 °C.
IR (KBr): 1/λ (cm⁻¹) = 2945, 1708 (CO Ester), 1466, 1406, 1216, 1119, 1098, 831, 777, 702, 696.
¹H-NMR (CDCl₃): δ (ppm) = 7.26 - 7.11 (m, 7 H, Ph +AA⁻), 6.99 - 6.94 (BB', 2 H), 4.14 (s, 2 H, CH₂), 3.65 (s, 3 H, OCH₃), 2.84 (s, 2 H, CH₂), 1.31 (s, 6 H, C(CH₃)₂).
¹³C-NMR (CDCl₃): δ (ppm) = 161.6 (CO), 140.3, 134.6, 134.0, 132.4, 132.1, 132.2, 128.5, 128.1, 127.6, 125.5, 117.8, 62.0 (CN₂), 50.7 (OCH₃), 42.4 (C-2), 40.3 (CH₂), 28.0 (C(CH₃)₂).
CHN: ber. C 72.72, H 5.84, N 3.69,
gef. C 72.57, H 5.78, N 3.64.

### b) 6-(4-Chlorphenyl)-2,3-dihydro-1-methoxy-2,2-dimethyl-7-phenyl-1H-pyrrolizin-5-yl]-carbonsäuremethylester

6-(4-Chlorphenyl)-2,3-dihydro-2,2-dimethyl-7-phenyl-1*H*-pyrrolizin-5-yl]-carbonsäuremethylester (Beispiel 1a, 4.0 g (10.5 mmol) wird mit 2.3 g (13.0 mmol) NBS in CCl₄ zum [1-Brom-6-(4-chlorphenyl)-2,3-dihydro-2,2-dimethyl-7-phenyl-1*H*-pyrrolizin-5-yl]-carbonsäuremethylester umgesetzt. Das Zwischenprodukt wird nicht isoliert, sondern durch Zusatz von 30 mL MeOH direkt umgesetzt. Nach wenigen Minuten scheidet sich ein Feststoff ab. Das Lösungsmittel wird abgesaugt und der Feststoff mit wenig Methanol gewaschen.

Ausbeute: 3.5 g (81 %), C₂₄H₂₄ClNO₃, 409.9 g/mol,
Smp.: 121 °C.
IR (KBr): 1/λ (cm⁻¹) = 2981, 2956, 2900, 1698 (CO Ester), 1455, 1438, 1312, 1217, 1135, 1083, 1012, 780, 703.
¹H-NMR (CDCl₃): δ (ppm) = 7.26 - 7.04 (m, 9 H, 2 Ar.), 4.19 - 4.14 (m, 3H, 1-H + 3-H₂), 3.66 (s, 3 H, COOCH₃), 3.07 (s, 3 H, 1-OCH₃), 1.32 (s, 3 H, 2-CH₃), 1.21 (s, 3 H, 2-CH₃).
¹³C-NMR (CDCl₃): δ (ppm) = 161.6 (COOCH₃), 139.3, 134.5, 133.6, 132.4, 131.8, 132.2, 129.4, 128.1, 127.6, 126.0, 121.2, 116.2, 83.2 (C-1), 60.1 (C-3), 57.5 (OCH₃), 50.9 (COOCH₃), 46.7 (C-2), 27.2, 20.5 (C(CH₃)2).
CHN: ber. C 70.32, H 5.90, N 3.42,
gef. C 70.04, H 5.70, N 3.36.

### Beispiel 2:

### 6-(4-Chlorphenyl)-2,3-dihydro-1-methoxy-2,2-dimethyl-7-phenyl-1H-pyrrolizin-5-yl-carbonsäure

6-(4-Chlorphenyl)-2,3-dihydro-1-methoxy-2,2-dimethyl-7-phenyl-1*H*pyrrolizin-5-yl]-carbonsäuremethylester (Beispiel 1b, 3.5 g, 8.5 mmol) werden in einer Lösung von 3.0 g (53.5 mmol) KOH in 40 mL MeOH 16 h zum Rückfluss erhitzt. Dann lässt man abkühlen und rührt in 400 mL Wasser ein, säuert mit konz. HCl auf pH 3 an und extrahiert dreimal mit je 100 mL Diethylether. Die Etherextrakte werden mit Wasser gewaschen und über Na₂SO₄ getrocknet. Man filtriert und engt bis zum Beginn der Kristallisation ein. Der farblose Feststoff wird abgesaugt und getrocknet.

Ausbeute: 2.9 g (86 %), C₂₃H₂₂ClNO₃, 395.9 g/mol,
Smp.: 195 °C.
IR (KBr): 1/λ (cm⁻¹) = 2958, 2895 (OH Säure), 1650 (CO Säure), 1516, 1492, 1469, 1316, 1136, 1107, 1091.
¹H-NMR ([D₆]-DMSO): δ (ppm) = 7.29 - 7.11 (m, 7 H, Ph + AA'), 7.04 - 7.00 (BB', 2 H), 4.26 (s, 1H, 1-H), 4.12 und 3.98 (m, 2 H, AB-System, 3-H₂, ²J = 12.0 Hz), 2.97 (s, 3 H, OCH₃), 1.22 (s, 3 H, 2-CH₃), 1.14 (s, 3 H, 2-CH₃).
¹³C-NMR ([D₆]-DMSO): δ (ppm) = 161.6 (CO), 138.6, 134.3, 134.1, 131.1, 130.2 , 132.5, 129.0, 128.1, 127.3, 125.9, 120.0, 116.5, 82.4 (C-1), 59.7 (C-3), 56.8 (OCH₃), 46.2 (C-2), 26.5, 20.3 (C(CH₃)₂).
CHN: ber. C 69.78, H 5.60, N 3.54,
gef. C 69.58, H 5.65, N 3.45.

### Beispiel 3:

### 6-(4-Chlorphenyl)-2,3-dihydro-1-hydroxy-2,2-dimethyl-7-phenyl-1H-pyrrolizin-5-yl-carbonsäure

6-(4-Chlorphenyl)-2,3-dihydro-1-methoxy-2,2-dimethyl-7-phenyl-1*H*-pyrrolizin-5-yl]-carbonsäure (Beispiel 2, 0.50 g, 1.26 mmol wird in 10 mL DMSO mit ca. 10% Wassergehalt gelöst und nach Zusatz von 1.5 mL konz. HCl 4 Tage bei Raumtemperatur gerührt. Dann versetzt man mit 30 mL Wasser. Das Kristallisat wird durch Absaugen vom Lösungsmittel befreit und anschließend in 20 mL Diethylether aufgenommen. Man wäscht die organische Phase einmal mit 20 mL Wasser und trocknet sie über Na₂SO₄. Nach Filtration wird das Lösungsmittel abrotiert. Der feste Rückstand wird durch Umkristallisieren aus Diethylether gereinigt.

Ausbeute: 0.10 g (21 %), C₂₂H₂₀ClNO₃, 381.9 g/mol,
Smp.: 204 °C.
IR (KBr): 1/λ (cm⁻¹)= 3510 (OH ), 2954, 1639 (CO Säure) , 1468, 1313, 1139, 1102, 1091, 696.
¹H-NMR ([D₆]-DMSO): δ (ppm) = 7.33 - 7.05 (m, 9 H, 2 Ar.), 5.55 (d, 1 H, OH, ³J = 8.3 Hz), 4.30 (d, 1 H, 1-H, ³J = 8.3 Hz), 4.06 (m, 2 H, 3-H₂), 1.17 (s, 3 H, 2-CH₃), 1.02 (s, 3 H, 2-CH₃).
¹³C-NMR ([D₆]-DMSO): δ (ppm) = 161.7 (CO), 141.5, 134.5, 134.2, 131.1, 130.2, 132.5, 128.6, 128.0, 127.4, 125.5, 118.4, 116.0, 74.1 (C-1), 59.5 (C-3), 46.0 (C-2), 26.4, 20.6 (C(CH₃)₂).
CHN: ber. C 69.20, H 5.28, N 3.67,
gef. C 68.70, H 5.42, N 3.51.

### Beispiel 4:

### 6-(4-Chlorphenyl)-2,3-dihydro-1-oxo-2,2-dimethyl-7-phenyl-1H-pyrrolizin-5-yl-carbonsäuremethylester (129)

6-(4-Chlorphenyl)-2,3-dihydro-2,2-dimethyl-7-phenyl-1*H*-pyrrolizin-5-yl]-carbonsäuremethylester (Beispiel 1a, 8.0 g, 21.1 mmol) und 9.4 g (53 mmol) NBS werden in 40 mL CCl₄ 3 h unter Rückfluss erhitzt. Dann setzt man 2.5 mL Wasser zu und kocht weitere 16 h unter Rückfluss. Die Reaktionsmischung wird auf ein Restvolumen von 5 mL eingeengt und mit 40 mL MeOH versetzt. Man kristallisiert bei 4 °C. Man saugt den hellen Feststoff vom Lösungsmittel ab und trocknet im Vakuum.

Ausbeute: 4.36 g (53 %), C₂₃H₂₀ClNO₃, 393.9 g/mol,
Smp.: 233 °C.
IR (KHr): 1/λ (cm⁻¹) = 2976, 2872, 1707, 1699 (CO Ester + Keton), 1464, 1313, 1238, 1147, 1105, 692.
¹H-NMR (CDCl₃): δ (ppm) = 7.30 - 7.10 (m, 9 H, 2 Ar.), 4.46 (s, 2 H, 3-H₂), 3.73 (s, 3 H, OCH₃), 1.39 (s, 6 H, 2-CH₃).
¹³C-NMR (CDCl₃): δ (ppm) = 196.1 (CO), 160.9 (CO), 133.7, 133.3, 132.4, 131.0, 130.2 , 132.1, 129.9, 128.0, 127.9, 127.3, 125.0, 122.0, 59.2 (C-3), 51.6 (OCH₃), 48.8 (C-2), 24.6 (C(CH₃)₂).

### Beispiel 5:

### 6-(4-Chlorphenyl)-2,3-dihydro-2,2-dimethyl-1-oxo-7-phenyl-1H-pyrrolizin-5-yl-carbonsäure

6-(4-Chlorphenyl)-2,3-dihydro-1-oxo-2,2-dimethyl-7-phenyl-1*H*-pyrrolizin-5-yl-carbonsäuremethylester (Beispiel 4, 4.4 g, 11.1 mmol) wird in einer Lösung von 3.0 g (53.5 mmol) KOH in 40 mL MeOH 2 h zum Rückfluss erhitzt. Dann lässt man abkühlen und rührt in 600 mL Wasser ein. Die trübe Lösung wird mit konzentrierter Salzsäure auf pH 3 gebracht. Der Niederschlag durch Filtration abgetrennt und im Vakuumexsikkator über Phosphorpentoxid getrocknet.

Ausbeute: 3.8 g (90 %), C₂₂H₁₈ClNO₃, 379.9 g/mol,
Smp.: 284 °C.
IR (KBr): 1/λ (cm⁻¹) = 2970, 1707 (CO Keton), 1656 (CO Säure),1518, 1476, 1316, 1302, 1148, 1101, 699.
¹H-NMR ([D₆]-DMSO): δ (ppm) = 7.35 - 7.31 (AA', 2 H), 7.20 - 7.10 (m, 7 H, Ph + BB'), 4.45 (s, 2 H, CH₂), 1.26 (s, 6 H, 2-CH₃).
¹³C-NMR ([D₆]-DMSO): δ (ppm) = 196.1 (C-1), 161.0 (COOH), 132.9, 132.3. 131.8, 131.3 , 132.4, 129.6, 127.8, 127.6, 127.0, 129.2, 123.4, 122.9 , 58.5 (C-3), 48.3 (C-2), 24.1 (C(CH₃)₂).
CHN: ber. C 69.56, H 4.78, N 3.69,
gef. C 69.06, H 4.81, N 3.53.

### Beispiel 6:

### 6-(4-Chlorphenyl)-2,3-dihydro-2,2-dimethyl-1-oxo-7-phenyl-1H-pyrrolizin

6-(4-Chlorphenyl)-2,3-dihydro-2,2-dimethyl-1-oxo-7-phenyl-1*H*pyrrolizin-5-yl-carbonsäure (Beispiel 5, 2.0 g, 5.3 mmol) wird 10 min zur Schmelze erhitzt (ca. 290 °C). Sobald die Gasentwicklung beendet ist, lässt man abkühlen und behandelt den Schmelzkuchen im Ultraschallbad mit MeOH (30 min). Dann wird das Lösungsmittel abgesaugt und der hellbraune Feststoff getrocknet.

Ausbeute: 1.31 g (74 %), C₂₁H₁₈ClNO, 335.8 g/mol,
Smp. 261 °C.
IR (KBr) : 1/λ (cm⁻¹) = 3116 (CH ), 2970 (CH ), 1684 (CO Keton), 1529, 1350, 1092, 1016, 821, 698, 534.
¹H-NMR (CDCl₃): δ (ppm) = 7.47 - 7.10 (m, 9 H, 2 Ar.), 7.09 (s, 1 H, 5-H), 4.14 (s, 2H, 3-H₂), 1.37 (s, 6H, 2-CH₃).
¹³C-NMR (CDCl₃): δ (ppm) = 194.6 (CO), 133.3, 132.5, 132.0, 129.9, 123.8 , 130.0, 129.8, 128.6, 128.1, 127.8, 121.9, 56.8 (C-3), 49.7 (C-2), 24.8 (C(CH₃)₂).
CHN: ber. C 75.11, H 5.40, N 4.17,
gef. C 73.51, H 5.27, N 4.09.

### Beispiel 7:

### 6-(4-Chlorphenyl)-2,3-dihydro-1-hydroxy-2,2-dimethyl-7-phenyl-1H-pyrrolizin

6-(4-Chlorphenyl)-2,3-dihydro-2,2-dimethyl-1-oxo-7-phenyl-1*H*pyrrolizin (Beispiel 6, 0.30 g, 0.9 mmol) wird in THF suspendiert. Man setzt 0.04 g (1.1 mmol) LiAlH₄ zu und erhitzt 0.5 h zum Rückfluss. Dann lässt man abkühlen und zersetzt überschüssiges Hydrid durch Zugabe von 10 mL gesättigter NaHCO₃-Lösung. Anschließend wird 10 mL 10 proz. NaOH zugesetzt und die organische Phase abgetrennt. Die wässrige Phase wird noch dreimal mit 10 mL Diethylether extrahiert. Die organischen Extrakte werden über Na₂SO₄ getrocknet, filtriert und einrotiert, Das Produkt schäumt im Vakuum auf. Es wird im Vakuum bei 40 °C von Restlösemittel befreit.

Ausbeute: 0.28 g (96 %), C₂₁H₂₀ClNO, 337.9 g/mol
IR (KBr) : 1/λ (cm⁻¹) = 3336 (b), 2958, 2870, 1601, 1525, 1486, 1409, 1173, 1092, 1032, 1012, 833, 767, 700.
¹H-NMR (CDCl₃): δ (ppm) = 7.35 - 7.12 (m, 9 H, 2 Ar.), 6.71 (s, 1 H, 5-H), 4.55 (d, 1 H, 1-H, ³J = 5.3 Hz), 3.96 und 3.64 (AB, 2 H, 3-H₂, ²J = 10.5 Hz), 1.76 (d, 1 H, 1-OH, ³J = 5.5 Hz), 1.30 (s, 3 H, 2-CH₃), 1.14 (s, 3 H, 2-CH₃).
¹³C-NMR (CDCl₃): δ (ppm) = 136.9, 135.1, 134.7, 131.2, 129.6, 128.9, 128.28, 128.26, 125.6, 125.7, 117.3, 114.4 (C-5), 75.4 (C-1), 57.5 (C-3), 47.8 (C-2), 26.6, 20.8 (C(CH₃)₂).
CHN: ber. C 74.67, H 5.97, N 4.15,
gef. C 74.06, H 5.99, N 3.97.

### Beispiel 8:

### 6-(4-Chlorphenyl)-2,3-dihydro-2,2-dimethyl-1-oxo-7-phenyl-1H-pyrrolizin-5-yl-carbaldehyd

6-(4-Chlorphenyl)-2,3-dihydro-2,2-dimethyl-1-oxo-7-phenyl-1*H*pyrrolizin (Beispiel 6, 0.30 g (0.9 mmol) wird in 1 mL DMF suspendiert. Die Suspension wird auf 5 °C gekühlt; dann setzt man 0.28 g (1.8 mmol) POCl₃ zu. Man rührt 30 min bei Raumtemperatur und erhitzt anschließend 16 h auf 80 °C. Die abgekühlte Reaktionsmischung wird mit 10 mL 10 proz. Natronlauge hydrolysiert und zweimal mit 20 mL Methylenchlorid extrahiert. Die organischen Extrakte wäscht man dreimal mit 40 mL Wasser, trocknet über Na₂SO₄, filtriert und rotiert ein. Das Rohprodukt wird aus Ethanol umkristallisiert.

Ausbeute: 250 mg (76 %), C₂₂H₁₈ClNO₂, 363.8 g/mol,
Smp. 185 °C.
IR (KBr): 1/λ (cm⁻¹) = 2962, 2868,1716 (CO Aldehyd), 1662 (CO Keton),1514, 1458, 1406, 1381, 1118, 1014, 845, 746, 723.
¹H-NMR (CDCl₃): δ (ppm) = 9.64 (s, 1 H, CHO), 7.38 - 7.19 (m, 9 H, 2 Ar.), 4.49 (s, 2 H, 3-H₂), 1.39 (s, 6 H, 2-CH₃).
¹³C-NMR (CDCl₃): δ (ppm) = 196.9 (CO), 181.7 (CHO), 136.8, 134.3, 130.5, 130.3, 129.3, 124.1 , 131.9, 129.7, 128.9, 128.2, 127.6, 58.6 (C-3), 49.2 (C-2), 24.6 (C(CH₃)₂).
CHN: ber. C 72.63, H 4.99, N 3.85,
gef. C 72.34, H 5.22, N 3.80.

### Beispiel 9:

### 1-Acetoxy-6-(4-chlorphenyl)-2,3-dihydro-2,2-dimethyl-7-phenyl-1H-pyrrolizin-5-yl-essigsäure tert-butylester

### a) 6-(4-Chlorphenyl)-2,3-dihydro-2,2-dimethyl-7-phenyl-1H-pyrrolizin-5-yl-essigsäure-tert-butylester

6-(4-Chlorphenyl)-2,3-dihydro-1-hydroxy-2,2-dimethyl-7-phenyl-1*H*pyrrolizin-5-yl]-essigsäure (ML3000, 10.0 g, 26.3 mmol), hergestellt nach Laufer et al. (J. Med. Chem. 1994, 37, 1894-1897) wird in 40 mL absolutem THF gelöst und mit 5.1 g (31.5 mmol) Carbonyldiimidazol versetzt. Man rührt 30 min bis das Imidazolid bei Raumtemperatur ausfällt, setzt dann 80 mL *tert*-Butanol zu und erhitzt am Wasserabscheider auf 100 °C. Sobald das THF vollständig überdestilliert ist, erhitzt man die Reaktionsmischung weitere 7 Tage auf 110 °C. Dann lässt man abkühlen und rührt mit 80 mL Diethylether oder MeOH aus. Das Kristallisat wird durch Absaugen vom Lösungsmittel befreit und getrocknet. Aus der Mutterlauge lässt sich durch Einrotieren und Fällen mit MeOH weiteres Kristallisat gewinnen.

Ausbeute: 8.0 g (70%), C₂₇H₃₀ClNO₂ 436.0 g/mol,
Smp.: 168 °C.
IR (KBr): 1/λ (cm⁻¹) = 2954, 2870,1728 (CO Ester), 1487, 1379, 1163, 1151, 1097, 822, 700. - ¹H-NMR (CDCl₃): δ (ppm) = 7.27 - 7.03 (m, 9 H, 2 Ar.), 3.75 (s, 2 H, CH₂), 3.41 (s, 2 H, CH₂), 2.84 (s, 2 H, CH₂), 1.47 (s, 9 H, (CCH₃)₃), 1.29 (s, 6 H, 2-CH₃).
¹³C-NMR (CDCl₃): δ (ppm) = 170.1 (CO), 136.1, 134.8, 133.9, 131.5, 131.6, 128.2, 128.1, 128.0, 124.6, 118.4, 114.6 , 81.2 (OC(CH₃)₃), 58.3 (CH₂), 43.3 (C-2), 40.5 (CH₂), 32.9 (CH₂), 28.1 (OC(CH₃)₃), 28.0 (C(CH₃)₂).
CHN: ber. C 74.38, H 6.94, N 3.21,
gef. C 73.99, H 6.90, N 3.21.

### b) 1-Brom-6-(4-chlorphenyl)-2,3-dihydro-2,2-dimethyl-7-phenyl-1H-pyrrolizin-5-yl-essigsäure-tert-butylester

6-(4-Chlorphenyl)-2,3-dihydro-2,2-dimethyl-7-phenyl-1*H*-pyrrolizin-5-yl-essigsäure-*tert*-butylester (Beispiel 9a, 5.0 g, 11.5 mmol) und NBS (2.2 g, 12.4 mmol) werden mit 50 mL CCl₄ und einer Spatelspitze AIBN versetzt. Die Reaktionsmischung wird 45 min auf 50 °C erhitzt. Nach Abkühlen wird das Succinimid durch Filtration abgetrennt und das Lösungsmittel im Vakuum entfernt.

Ausbeute: 5.6 g (95 %) C₂₇H₂₉BrClNO₂; 514.9 g/mol.
IR (KBr): 1/λ (cm⁻¹) = 2968, 2931 (s, aliphat. CH ), 1730 (s,breit, CO , Ester), 1466, 1367, 1147, 1099, 833, 787, 770 (s, C-Br ).
¹H-NMR (CDCl₃): δ (ppm) = 7.30 - 7.12 (m, 7 H, Ph + AA'), 7.11 - 7.06 (BB', 2 H), 5.15 (s, 1 H, 1-H), 3.81 (s, breit, 2 H, 3-H₂), 3.41 (s, 2 H, CH₂COOR), 1.48 (s, 9 H, C(CH₃)₃), 1.35 (s, breit, 6 H, 2 CH₃).
¹³C-NMR (CDCl₃): δ (ppm) = 169.5 (CO), 134.8, 133.9, 133.5, 132.0,131.7, 128.9, 128.3, 128.1, 125.6, 124.3, 121.7, 118.0, 81.6 (OC(CH₃)₃), 60.8 (C-1), 55.4 (CH₂), 49.4 (C-3), 32.9 (CH₂), 28.8 (OC(CH₃)₃), 25.9 (C(CH₃)₂).

### c) 1-Acetoxy-6-(4-chlorphenyl)-2,3-dihydro-2,2-dimethyl-7-phenyl-1H-pyrrolizin-5-yl-essigsäure-tert-butylester

1-Brom-6-(4-chlorphenyl)-2,3-dihydro-2,2-dimethyl-7-phenyl-1*H*pyrrolizin-5-yl-essigsäure-*tert*-butylester (Beispiel 9b, 1.15 g, 2.23 mmol) wird mit 0.5 g Kaliumacetat, 10 mL absolutem Diethylether und 3 Tropfen Essigsäure versetzt und 3 h unter Rückfluss erhitzt. Dann lässt man abkühlen, filtriert die anorganischen Salze ab und rotiert die Etherlösung ein. Bei einem Restvolumen von ca. 5 mL werden 10 mL Hexan zugesetzt. Nach Anreiben fällt das Produkt aus und wird durch Absaugen des Lösungsmittels abgetrennt.

Ausbeute 0.74 g (65 %) C₂₉H₃₂ClNO₄; 494.0 g/mol,
Smp.: 81 °C.
IR (KBr): 1/λ (cm⁻¹) = 2972, 1728 (CO Ester), 1368, 1246, 1149, 1014, 833, 708.
¹H-NMR (CDCl₃): δ (ppm) = 7.24 - 6.99 (m, 9 H, 2.Ar.), 5.92 (s, 1 H, 1-H), 3.92 und 3.75 (2 H, AB, 3-H₂, ²J = 10.4 Hz), 3.43 (s, 2 H, CH₂COOR), 2.09 (s, 3 H, OCH₃), 1.48 (s, 9 H, OC(CH₃)₃), 1.25 (s, 3 H, 2-CH₃), 1.21 (s, 3 H, 2-CH₃).
¹³C-NMR (CDCl₃): δ (ppm) = 170.3 (CO), 169.7 (CO), 134.7, 134.1, 131.8, 130.8 , 131.7, 128.6, 128.2, 128.0, 125.5, 124.3, 120.1, 118.6 , 81.5, (OC(CH₃)₃), 76.3 (C-1), 56.5 (C-3), 47.6 (C-2), 32.8 (CH₂COOR), 28.1 (OC(CH₃)₃), 26.6 (OCH₃), 21.0, 20.9 (CH₃).
CHN: ber. C 70.50, H 6.53, N 2.84,
gef. C 70.83, H 6.71, N 2.81.

### Beispiel 10:

### 1-Acetoxy-6-(4-chlorphenyl)-2,3-dihydro-2,2-dimethyl-7-phenyl-1H-pyrrolizin-5-yl]-2-oxoessigsäureethylester

### a) 1-Brom-6-(4-chlorphenyl)-2,3-dihydro-2,2-dimethyl-7-phenyl-1H-pyrrolizin-5-yl-2-oxo-essigsäureethylester

6-(4-Chlorphenyl)-2,3-dihydro-2,2-dimethyl-7-phenyl-1*H*-pyrrolizin-5-yl]-2-oxoessigsäureethylester (9.82 g, 23.3 mmol), hergestellt nach Lit. wird in 50 mL CCl₄ gelöst. Dann setzt man 4.70 g (26.4 mmol) NBS sowie eine Spatelspitze Azoisobuttersäurenitril (AIBN) zu und erhitzt 4 h zum Rückfluss. Nachdem die Reaktionsmischung abgekühlt ist, wird das Succinimid abfiltriert und die klare Lösung einrotiert. Der Rückstand kristallisiert aus Diisopropylether.

Ausbeute: 8.9 g (76 %), C₂₅H₂₃BrClNO₃, 500.8 g/mol.
Smp 167 °C.
IR (KBr): 1/λ (cm⁻¹) = 2966 (CH) , 1740 (CO Ester), 1630 (COα - Keton), 1446, 1433, 1257, 1068, 1018, 851, 696.
¹H-NMR (CDCl₃): δ (ppm) = 7.30 - 7.13 (m, 9 H, 2 Ar.), 5.00 (s, 1 H, 1-H), 4.34 und 4.14 (2 H, AB, 3-CH₂, ²J = 12.5 Hz), 3.64 (m, 2 H, ABX₃, OCH₂, ²J = 10.8 Hz, ³J = 7.3 Hz), 1.51 (s, 3 H, 2-CH₃), 1.28 (s, 3 H, 2-CH₃), 1.08 (t, 3 H, OCH₂CH₃, ³J = 7.2 Hz).
¹³C-NMR (CDCl₃): δ (ppm) = 177.4, 164.1 (CO), 143.3, 135.4, 134.0, 132.4, 131.7 , 132.2, 129.0, 128.3, 128.2, 126.8, 122.9. 120.5 , 61.9 (CH₂), 59.0 (CH₂), 54.9 (C-1), 48.3 (C-2), 26.4, 24.9 (C(CH₃)₂), 13.5 (OCH₂CH₃).
CHN: ber. C 59.96, H 4.63, N 2.80,
gef. C 59.95, H 4.64, N 2.74.

### b) 1-Acetoxy-6-(4-chlorphenyl)-2,3-dihydro-2,2-dimethyl-7-phenyl-1H-pyrrolizin-5-yl-2-oxoessigsäureethylester

1-Brom-6-(4-chlorphenyl)-2,3-dihydro-2,2-dimethyl-7-phenyl-1*H*pyrrolizin-5-yl-2-oxo-essigsäureethylester (Beispiel 10a, 10.0 g 20 mmol) wird in 50 mL DMF gelöst. Nach Zugabe von Kaliumacetat (5.0 g, 51 mmol) wird 2 h auf 80 °C erhitzt. Die abgekühlte Reaktionsmischung wird auf Eis gegossen und mit 200 mL Diethylether extrahiert. Die Etherphase wird abgetrennt, zweimal mit 150 mL Wasser gewaschen, über Na₂SO₄ getrocknet, filtriert und einrotiert.
Man erhält ein Gemisch von 1-Acetoxy-6-(4-chlorphenyl)-2,3-dihydro-2,2-dimethyl-7-phenyl-1*H*-pyrrolizin-5-yl-2-oxoessigsäureethylester und 2-[6-(4-Chlorphenyl)-2,3-dihydro-1-hydroxy-2,2-dimethyl-7-phenyl-1*H*-pyrrolizin-5-yl-2-oxoessigsäureethylester.

Ausbeute: 6.9 g (75 %) eines Gemisches mit wechselnden Anteilen der beiden Komponenten). Trennung der Komponenten mittels SC (Kieselgel 60, Ether/Cyclohexan 1:1) liefert:
2-[1-Acetoxy-6-(4-chlorphenyl)-2,3-dihydro-2,2-dimethyl-7-phenyl-1*H*-pyrrolizin-5-yl-2-oxoessigsäureethylester
C₂₇H₂₆ClNO₅, 480.0 g/mol:
Smp.: 142 °C
IR (KBr): 1/λ (cm⁻¹) = 2970 (CH ), 1747 (CO Ester), 1624 (COα Keton), 1448, 1427, 1227, 1084, 1018, 733, 696.
¹H-NMR (CDCl₃): δ (ppm) = 7.29 - 6.91 (m, 9 H, 2 Ar.), 5.95 (s, 1 H, 1-H), 4.32 und 4.24 (2 H, AB, 3-H₂, ²J = 12.4 Hz), 3.70 - 3.55 (m, 2 H, ABX₃, OCH₂), 2.03 (s, 3 H, OCH₃), 1.30 (s, 3 H, 2-CH₃), 1.22 (s, 3 H, 2-CH₃), 1.07 (t, 3 H, OCH₂CH₃, ³J = 7.1 Hz).
¹³C-NMR (CDCl₃): δ (ppm) = 177.4 (CO α-Keton), 169.8 (CO), 164.1 (CO), 140.8, 135.5, 133.9, 132.3, 131.8, 128.9, 128.3, 121.3 , 132.1, 128.8, 128.2, 128.1, 126.7, 75.0 (C-1), 61.8 (CH₂), 60.4 (CH₂), 46.7 (C-2), 26.4 (COCH₃), 20.8, 20.6 (CH₃), 13.5 (OCH₂CH₃).
CHN: ber. C 67.57, H 5.46, N 2.92,
gef. C 67.48, H 5.48, N 2.89.

### Beispiel 11:

### 6-(4-Chlorphenyl)-2,3-dihydro-1-hydroxy-2,2-dimethyl-7-phenyl-1H-pyrrolizin-5-yl-2-oxoessigsäureethylester

nach SC aus Beispiel 10b:
C₂₅H₂₄ClNO₄, 438.0 g/mol.
Smp.: 193 °C.
IR (KBr): 1/λ (cm⁻¹) = 3520 (OH ), 2958 (CH), 1730 (CO Ester), 1622 (CO α-Keton),1450, 1425, 1269, 1076, 1016, 737, 700.
¹H-NMR (CDCl₃): δ (ppm) = 7.29 - 7.12 (m, 9 H, 2 Ar.), 4.64 (d, 1 H, 1-H, ³J = 5.8 Hz), 4.29 und 4.23 (2H, AB, 3-H₂, ²J = 9.8 Hz), 3.75 - 3.50 (m, 2 H, ABX₃, OCH₂), 1.96 (d, 1 H, OH, ³J = 5.8 Hz), 1.32 (s, 3 H, 2-CH₃), 1.20 (s, 3 H, 2-CH₃), 1.07 (t, 3 H, OCH₂CH₃, ³J = 7.2 Hz).
¹³C-NMR (CDCl₃): δ (ppm) = 177.5 (CO α-Keton), 164.3 (CO Ester), 144.6, 135.5, 133.9, 132.8, 132.0 , 132.2, 129.0, 128.4, 128.2, 126.6, 122.0, 120.8 , 75.1 (C-1), 61.8 (CH₂), 60.0 (CH₂), 46.9 (C-2), 26.4 (C(CH₃)₂), 20.5 (C(CH₃)₂), 13.5 (OCH₂CH₃).

### Beispiel 12:

### 6-(4-Chlorphenyl)-2,3-dihydro-1-hydroxy-2,2-dimethyl-7-phenyl-1H-pyrrolizin-5-yl-essigsäure

1-Acetoxy-6-(4-chlorphenyl)-2,3-dihydro-2,2-dimethyl-7-phenyl-1*H*pyrrolizin-5-yl-essigsäure-*tert*-butylester (Beispiel 9c, 1.00 g, 2.0 mmol) wird in 10 mL MeOH gelöst. Man setzt 0.5 g KOH zu und erhitzt unter Rückfluss bis sich der intermediär gebildete Niederschlag aufgelöst hat. Dann lässt man abkühlen und rührt in 600 mL Wasser ein. Die trübe Lösung wird mit konz. HCl auf pH 3 gebracht. Es wird dreimal mit je 100 mL Diethylether extrahiert. Die Etherextrakte werden zweimal mit Wasser gewaschen und über Na₂SO₄ getrocknet, filtriert und einrotiert. Man erhält ein Gemisch aus 6-(4-Chlorphenyl)-2,3-dihydro-1-hydroxy-2,2-dimethyl-7-phenyl-1*H*pyrrolizin-5-yl-essigsäure und 2-[6-(4-Chlorphenyl)-2,3-dihydro-1-methoxy-2,2-dimethyl-7-phenyl-1*H*-pyrrolizin-5-yl]-essigsäure (vgl. Beispiel 16).

Alternativsynchese (6-(4-Chlorphenyl)-2,3-dihydro-1-hydroxy-2,2-dimethyl-7-phenyl-1*H*-pyrrolizin-5-yl-essigsäure)
Ein Produktgemisch welches ein 1:2 Gemisch an 2-[1-Acetoxy-6-(4-chlorphenyl)-2,3-dihydro-2,2-dimethyl-7-phenyl-1*H*-pyrrolizin-5-yl]-2-oxoessigsäureethylester und 2-[6-(4-Chlorphenyl)-2,3-dihydro-1-hydroxy-2,2-dimethyl-7-phenyl-1*H*-pyrrolizin-5-yl]-2-oxoessigsäureethylester enthält (aus Beispiel 10b, 1.50 g, 3.31 mmol) wird in 15 mL Diethylenglykol suspendiert, mit 1.3 mL Hydrazinhydrat (80 proz.) versetzt und 1 h auf 80 °C erhitzt. Dann lässt man abkühlen und setzt 2.5 g KOH zu. Anschließend wird 1 h auf 130 °C und 2 h auf 160 °C erhitzt. Nach dem Abkühlen wird mit 30 mL Wasser verdünnt und mit konz. HCl auf pH 2 gebracht und dreimal mit 30 mL Diethylether extrahiert. Die organischen Extrakte wäscht man zweimal mit 50 mL Wasser, trocknet über Na₂SO₄, filtriert und rotiert bis auf ein Restvolumen von 50 mL ein. Dann setzt man ca. 20 mL Hexan zu und dampft bis zur beginnenden Kristallisation ein. Das Kristallisat wird durch Absaugen abgetrennt, die Lösung weiter eingeengt. Auf diese Weise erhält man mehrere Kristallisatfraktionen. Die letzten KrisLallisate enthalten die gesuchte Verbindung.

Ausbeute: 0.07 g (6 %) C₂₃H₂₂ClNO₃, 395.9 g/mol .
IR (KBr): 1/λ (cm⁻¹) = 3421 (OH), 2958, 2926, 2870 (OH), 1730 (CO Säure), 1485, 1090, 1012, 831, 700.
¹H-NMR (CDCl₃): δ (ppm) = 7.28 - 7.06 (m, 9 H, 2 Ar.), 4.61 (s, 1 H, 1-H), 3.91, 3.68 (2 H, AB, 3-H₂, ²J = 10.4 Hz), 3.58 (2 H, AB, CH₂COOH), 1.31 (s, 3 H, 2-CH₃), 1.15 (s, 3 H, 2-CH₃).

### Beispiel 13:

### 6-(4-Chlorphenyl)-2,3-dihydro-1-methoxy-2,2-dimethyl-7-phenyl-1H-pyrrolizin-5-yl-essigsäure-tert-butylester

1-Brom-6-(4-chlorphenyl)-2,3-dihydro-2,2-dimethyl-7-phenyl-1*H*pyrrolizin-5-yl]-essigsäure-*tert*-butylester (Beispiel 9b, 1.15 g, 2.23 mmol) wird in 10 mL MeOH gelöst. Nach wenigen Minuten scheidec sich ein Feststoff ab. Das Lösungsmittel wird abgesaugt und der Feststoff mit wenig MeOH gewaschen.

Ausbeute: 0.73 g (70 %), C₂₈H₃₂ClNO₃, 466.0 g/mol,
Smp.: 140 °C.
IR (KBr): 1/λ (cm⁻¹) = 2881, 1728 (CO Ester), 1153, 1369, 1342, 1214, 1089, 827, 746, 698. - ¹H-NMR (CDCl₃) : δ (ppm) = 7.25 - 7.08 (m, 9 H, 2 Ar.), 4.22 (s, 1 H, 1-H), 3.88, 3.65 (2 H, AB, 3-H₂, ²J = 10.3 Hz), 3.43 (s, 2 H, CH₂COOR), 3.09 (s, 3 H, OCH₃), 1.45 (s, 9 H, OC(CH₃)₃), 1.32 (s, 3 H, 2-CH₃), 1.20 (s, 3 H, 2-CH₃).
¹³C-NMR (CDCl₃) : δ (ppm) = 169.7 (CO), 135.9, 134.5, 133.3, 131.5 , 131.7, 129.1, 128.1, 128.0, 125.3, 123.7, 119.3, 118.9 , 83.5 (C-1), 81.3 (OC(CH₃)₃), 56.8 (OCH₃), 56.4 (C-3), 47.7 (C-2), 32.9 (CH₂COOR), 28.0 (OC(CH₃)₃), 27.3, 20.8 (C(CH₃)₂).
CHN: ber. C 72.17, H 6.92, N 3.01,
gef. C 71.38, H 6.85, N 2.99.

### Beispiel 14:

### 6-(4-Chlorphenyl)-2,3-dihydro-1-methoxy-2,2-dimethyl-7-phenyl-1H-pyrrolizin-5-yl-essigsäure

6-(4-Chlorphenyl)-2,3-dihydro-1-methoxy-2,2-dimethyl-7-phenyl-1*H*pyrrolizin-5-yl-essigsäure-*tert*-butylester (Beispiel 13, 1.00 g, 2.14 mmol) wird in 10 mL MeOH suspendiert. Man setzt 0.5 g KOH zu und erhitzt 2 h zum Rückfluss. Dann lässt man abkühlen und rührt in 600 mL Wasser ein. Die trübe Lösung wird mit konz. HCl auf pH 3 gebracht. Es wird dreimal mit je 100 mL Diethylether extrahiert. Die Etherextrakte werden zweimal mit Wasser gewaschen und über Na₂SO₄ getrocknet. Man filtriert und rotiert bis zum Beginn der Kristallisation ein. Der farblose Feststoff wird durch Absaugen vom restlichen Lösungsmittel befreit und getrocknet.

Ausbeute: 0.66 g (75 %), C₂₄H₂₄ClNO₃, 409.9 g/mol,
Smp.: 168 °C.
IR (KBr): 1/λ (cm⁻¹) = 3034 (CH), 2966, 2884 (OH), 1716 (CO Säure), 1092,1456, 1225, 1206, 1016, 697.
¹H-NMR ([D₆]-DMSO): δ (ppm) = 7.34 - 7.28 (m, 2 H, AA'), 7.25 - 7.02 (m, 7 H, Ph + BB'), 4.23 (s, 1 H, 1-H), 3.71 (s, 2 H, CH₂COOH), 3.55 und 3.41 (2 H, AB, 3-H₂, ²J = 16.8 Hz), 2.92 (s, 3 H, OCH₃), 1.21 (s, 3 H, 2-CH₃), 1.12 (s, 3 H, 2-CH₃).
¹³C-NMR (CDCl₃/[D₆]-DMSO): δ (ppm) = 172.4 (CO), 135.9, 134.4, 133.4, 131.2 , 131.6, 129.0, 128.0, 127.96, 125.2,, 123.3, 119.3, 118.6 , 83.4 (C-1), 56.9 (OCH₃), 56.4 (C-3), 47.5 (C-2), 31.4 (CH₂COOH), 27.3, 20.8 (C(CH₃)₃).
CHN: ber. C 70.33, H 5.90, N 3.42,
gef. C 69.67, H 5.90, N 3.36.

### Beispiel 15:

### 6-(4-Chlorphenyl)-2,3-dihydro-1-hydroxy-2,2,5-trimethyl-7-phenyl-1H-pyrrolizin

6-(4-Chlorphenyl)-2,3-dihydro-1-methoxy-2,2-dimethyl-7-phenyl-1*H*pyrrolizin-5-yl-essigsäure (Beispiel 14, 0.50 g, 1.22 mmol) wird in einer Mischung aus 5 mL DMSO und 1 mL Wasser suspendiert und 4 h auf 90 - 100 °C erhitzt. Nach Abkühlen der Reaktionsmischung setzt man 30 mL Diethylether zu, trennt die wässrige Phase ab und wäscht die Etherlösung dreimal mit 50 mL Wasser. Nach Trocknen über Na₂SO₄ und Filtration wird im Vakuum eingeengt. Der Rückstand wird aus Cyclohexan kristallisiert.

Ausbeute: 0.26 g (60 %), C₂₂H₂₂ClNO, 351.9 g/mol,
Smp.: 133 °C.
IR (KBr): 1/λ (cm⁻¹) = 3302 (OH), 2956, 2906,1486, 1381, 1054, 999, 831, 732, 699.
¹H-NMR (CDCl₃): δ (ppm) = 7.26 - 7.11 (m, 7 H, Ph + AA'), 7.09 - 7.05 (2 H, BB'), 4.57 (d, 1 H, 1-H, ³J = 5.1 Hz), 3.84 und 3.59 (2 H, AB, 3-H₂, ²J = 10.4 Hz), 2.19 (s, 3 H, 5-CH₃), 1.74 (d, 1 H, OH, ³J = 5.1 Hz), 1.31 (s, 3 H, 2-CH₃), 1.15 (s, 3 H, 2-CH₃).
¹³C-NMR ([D₆]-DMSO): δ (ppm) = 136.0, 135.7, 135.3, 130.0, 131.6, 128.3, 128.2, 128.1, 124.8, 121.6, 120.3, 115.8, 74.8 (C-1), 55.3 (C-3), 47.0 (C-2), 26.9 (C(CH₃)₂), 21.2 (C(CH₃)₂), 10.4 (CH₃-5).
MS (ES⁺, 35 V), *m*/*z* (% rel. Int.), 352 (11, (M+H)⁺), 334 (100). CHN: ber. C 75.09, H 6.30, N 3.98,
gef. C 75.21, H 6.37, N 4.18.

### Beispiel 16:

### 6-(4-Chlorphenyl)-2,3-dihydro-1-methoxy-2,2,5-trimethyl-7-phenyl-1H-pyrrolizin

6-(4-Chlorphenyl)-2,3-dihydro-1-hydroxy-2,2,5-trimethyl-7-phenyl-1*H*-pyrrolizin (Beispiel 15, 0.38 g, 1.08 mmol) wird in 10 mL MeOH gelöst. Die klare Lösung wird kurz mit HCl-Gas behandelt, bis ein heller Niederschlag ausfällt. Dann wird mit 10 mL MeOH verdünnt und der Feststoff durch Absaugen des Lösungsmittels abgetrennt. Man wäscht noch mehrmals mit MeOH nach und lässt an der Luft trocknen.

Ausbeute: 0.30 g (76 %), C₂₃H₂₄ClNO, 365.9 g/mol,
Smp.: 179 °C.
IR (KBr): 1/λ (cm⁻¹) = 2980, 2935, 2878, 1484, 1463, 1380, 1317, 1181, 1087, 819, 766, 750, 696.
¹H-NMR (CDCl₃): δ (ppm) = 7.25 - 7.11 (m, 7 H, Ph + AA'), 7.07 - 7.02 (2 H, BB'), 4.20 (s, 1 H, 1-H), 3.82 und 3.55 (2 H, AB, 3-H₂), 3.03 (s, 3 H, OCH₃), 2.20 (s, 3 H, 5-CH₃), 1.31 (s, 3 H, 2-CH₃), 1.21 (s, 3 H, 2-CH₃).
¹³C-NMR (CDCl₃) : δ (ppm) = 136.3, 135.1, 132.1, 130.9 , 131.5, 129.1, 128.0, 125.2, 122.2, 121.5, 118.8 , 83.5 (C-1), 56.8 (OCH₃), 55.9 (C-3), 47.4 (C-2), 27.5, 20.9 (C(CH₃)₂), 10.7 (CH₃-5)
CHN: ber. C 75.48, H 6.61, N 3.83,
gef. C 75.05, H 6.55, N 3.80.

### Beispiel 17:

### 6-(4-Chlorphenyl)-2,3-dihydro-2,2-dimethyl-1-oxo-7-phenyl-1H-pyrrolizin-5-yl]-essigsäure-tert-butylester

6-(4-Chlorphenyl)-2,3-dihydro-2,2-dimethyl-7-phenyl-1*H*-pyrrolizin-5-yl-essigsäure-tert-butylester (Beispiel 9a, 15.0 g, 34.4 mmol) und NBS (13.5 g, 75.8 mmol) werden in 75 mL CCl₄ suspendiert. Man setzt 5 mL Wasser zu und erhitzt 2.5 h auf 70 °C. Die abgekühlte Reaktionsmischung wird über Na₂SO₄ getrocknet, filtriert und das Lösungsmittel abrotiert. Der Rückstand kristallisiert bei 5 °C aus MeOH. Das Lösungsmittel wird abgesaugt und das Kristallisat mit eiskaltem MeOH gewaschen.

Ausbeute: 9.0 g (58%). C₂₇H₂₈ClNO₃; 450.0 g/mol),
Smp.: 180 °C.
IR (KBr): 1/λ (cm⁻¹) = 1728 (CO Ester), 1686 (CO Keton), 1541, 1365, 1294, 1144, 1095, 1014, 833, 694.
¹H-NMR (CDCl₃): δ (ppm) = 7.40 - 7.36 (AA', 2 H), 7.32 - 7.19 (m, 5 H, Ph), 7.14 - 7.09 (BB', 2 H), 4.10 (s, 2 H, CH₂), 3.50 (s, 2 H, CH₂), 1.47 (s, 9 H, OC(CH₃)₃,), 1.38 (s, 6 H, 2-CH₃).
¹³C-NMR (CDCl₃): δ (ppm) = 194.3 (CO), 168.3 (CO) , 133.0, 132.8, 132.1, 131.7, 129.7, 128.7, 127.9, 127.0, 128.5, 127.5, 124.7, 82.2 (OC(CH₃)₃), 55.4 (CH₂), 49.6 (C-2), 32.5 (C-3), 28.0 (OC(CH₃)₃), 24.9 (C(CH₃)₂).
CHN: ber. C 72.07, H 6.27, N 3.11,
gef. C 71.29, H 6.27, N 2.97.

### Beispiel 18:

### 6-(4-Chlorphenyl)-2,3-dihydro-2,2-dimethyl-1-oxo-7-phenyl-1H-pyrrolizin-5-yl-essigsäure

6-(4-Chlorphenyl)-2,3-dihydro-2,2-dimethyl-1-oxo-7-phenyl-1*H*pyrrolizin-5-yl]-essigsäure-*tert*-butylester (Beispiel 17, 14.0 g, 31.1 mmol) wird zu einer Lösung von 15.0 g KOH in 180 mL MeOH gegeben und erhitzt 2 h zum Rückfluss, lässt abkühlen und rührt in 600 mL Wasser ein. Die Lösung wird mit konz. HCl auf pH 3 gebracht und mit je 100 mL Diethylether extrahiert. Der Etherextrakte wird mit Wasser gewaschen und über Natriumsulfat getrocknet. Man filtriert und reduziert das Volumen im Vakuum bis zum Beginn der Kristallisation. Der Feststoff wird gesammelt und getrocknet.

Ausbeute: 10.6 g (86 %), C₂₃H₂₀ClNO₃; 393.9 g/mol,
Smp.: 234.3 °C.
IR (KBr): 1/λ (cm⁻¹) = 2968, 2926 (CH), 1743 (CO Säure), 1650 (CO Keton), 1534, 1364, 1330, 1172, 1107, 696.
¹H-NMR ([D₆]-DMSO): δ (ppm) = 7.43 - 7.38 (AA', 2 H), 7.30 - 7.13 (m, 5 H, Ph), 7.13 - 7.08 (BB', 2 H), 4.14 (s, 2 H, CH₂), 3.64 (s, 2 H, CH₂), 1.25 (s, 6 H, 2-CH₃).
¹³C-NMR ([D₆]-DMSO): δ (ppm) = 194.1 (CO) 170.5 (CO), 132.9, 132.1, 131.7 , 131.65, 129.3, 128.6, 127.9, 126.8, 128.4, 127.3, 125.6, 122.9, 54.6 (CH₂), 48.9 (C-2), 30.6 (CH₂), 24.4 (C(CH₃)₂).
CHN: ber. C 70.14, H 5.12, N 3.56,
gef. C 70.09, H 5.09, N 3.65.

### Beispiel 19

### 6-(4-Chlorphenyl)-2,3-dihydro-2,2,5-trimethyl-1-oxo-7-phenyl-1H-pyrrolizin

6-(4-Chlorphenyl)-2,3-dihydro-2,2-dimethyl-1-oxo-7-phenyl-1*H*pyrrolizin-5-yl-essigsäure (Beispiel 18, 0.60 g, 1.52 mmol) wird 15 min auf 245 °C erhitzt. Die abgekühlte Schmelze wird gemahlen und mit 10 mL Diethylether extrahiert. Das Lösungsmittel wird abgesaugt und das Kristallisat getrocknet.

Ausbeute: 420 mg (79 %). C₂₂H₂₀ClNO, 349.9 g/mol,
Smp.: 227 °C.
IR (KBr) : 1/λ (cm⁻¹) = 2966, 2866, 1682 (CO), 1537, 1461, 1399, 1359, 1322, 1125, 1089, 753, 695.
¹H-NMR (CDCl₃): δ (ppm) = 7.41 - 7.36 (AA', 2 H), 7.31 - 7.18 (m, 5 H, Ph), 7.11 - 7.06 (BB', 2 H), 4.01 (s, 2 H, CH₂), 2.25 (s, 3 H, CH₃), 1.38 (s, 6 H, 2-CH₃).
¹³C-NMR (CDCl₃): δ (ppm) = 193.8 (CO), 133.4, 132.5, 132.2, 130.9, 131.6, 129.7, 128.6, 127.9, 126.9, 127.2, 125.6, 124.9, 55.0 (C-3), 49.5 (C-2), 25.0 (C(CH₃)₂), 10.5 (C=C-CH₃).
CHN: ber. C 75.53, H 5.76, N 4.00,
gef. C 75.58, H 5.84, N 4.13.

### Beispiel 20:

### a) 2-[6-(4-Chlorphenyl)-2,3-dihydro-2,2-dimethyl-7-phenyl-1H-pyrrolizin-5-yl]-2-hydroxyessigsäureethylester

6-(4-Chlorphenyl)-2,3-dihydro-2,2-dimethyl-7-phenyl-1*H-*pyrrolizin-5-yl]-2-oxoessigsäureethylester (1.0 g, 2.4 mmol), hergestellt nach Laufer et al. (J. Med. Chem. 1994, 37, 1894-1897) wird in 10 mL THF gelöst. Dann fügt man eine Lösung von 0.03 g NaBH₄ in 3 mL Wasser zu und rührt das 2-Phasen-System 1 h bei Raumtemperatur. Anschließend wird mit 3 mL konz. NH₃-Lösung versetzt und nochmals 1 h gerührt. Die organische Phase wird abgetrennt, mit ca. 80 mL Wasser verdünnt und zweimal mit 20 mL Diethylether extrahiert. Die Etherphase wird dreimal mit 100 mL Wasser gewaschen, über Na₂SO₄ getrocknet und nicht ganz zur Trockene einrotiert. Das Produkt 20a kristallisiert nach Zugabe von 10 mL Hexan.

Ausbeute: 0.86 g (86 %), C₂₅H₂₆ClNO₃, 423.9 g/mol,
Smp.: 117 °C.
IR (KBr): 1/λ (cm⁻¹) = 3456 (OH), 2956, 2870, 1736 (CO Ester), 1603, 1448, 1213, 1065, 1014, 698.
¹H-NMR ([D₆]-DMSO): δ (ppm) = 7.38 - 7.34 (AA', 2 H), 7.19 - 7.00 (m, 5 H, Ph), 6.99 - 6.95 (BB', 2 H), 5.93 (d, 1 H, OH, ³J = 4.2 Hz), 4.96 (d, 1 H, CH, ³J = 4.4 Hz), 4.20 - 4.00 (m, 2 H, OCH₂), 3.84 und 3.67 (2 H, AB, 3-H₂, ²J = 11.0 Hz), 2.82 und 2.68 (2 H, AB, 1-H₂, ²J = 15.4 Hz), 1.24 (s, 3 H, 2-CH₃), 1.17 (s, 3 H, 2-CH₃), 1.16 (t, 3 H, OCH₂CH₃, ³J = 7.1 Hz).
¹³C-NMR ([D₆]-DMSO): δ (ppm) = 171.6 (CO), 135.5, 134.9, 134.3, 130.7 , 131.7, 128.1, 128.0, 127.7, 124.6, 122.8, 122.7, 113.6 , 64.8 (CHOH), 60.7 (CH₂), 58.8 (CH₂), 42.8 (C-2), 39.4 (CH₂), 27.4 (CH₃), 27.1 (CH₃), 14.0 (OCH₂CH₃).
CHN: ber. C 70.83, H 6.18, N 3.30,
gef. C 71.24, H 6.38, N 3.07.

### b) 2-[6-(4-Chlorphenyl)-2,3-dihydro-2,2-dimethyl-7-phenyl-1H-pyrrolizin-5-yl]-2-hydroxyessigsäure

Kaliumhydroxid (0.50 g ) wird in 10 ml 95 proz. Ethanol gelöst. Man setzt 6-(4-Chlorphenyl)-2,3-dihydro-2,2-dimethyl-7-phenyl-1*H* pyrrolizin-5-yl]-2-hydroxy-essigsäureethylester (0.40 g , 0.66 mmol) zu und rührt 16 h bei Raumtemperatur. Anschließend wird das Lösungsmittel abrotiert und der Rückstand zunächst mit Diethylether ausgelaugt und gewaschen, dann mit 100 ml Wasser versetzt. Die Lösung wird mit 3 M Salzsäure auf pH 4 angesäuert. Die sich abscheidende weiße Säure wird rasch in 20 ml Diethylether aufgenommen. Die organische Phase wird abgetrennt, mit 20 ml Wasser gewaschen, über Natriumsulfat getrocknet, filtriert und das Lösungsmittel abgedampft.

Ausbeute: 0.18 g (68 %), C₂₃H₂₂ClNO₃, 397.9 g/mol
IR (KBr): 1/λ = 3402 (s, br, OH), 2956, 2870 (s, aliphat. + COOH), 1718 (s, CO), 1603, 1099, 1059, 1012, 837, 696.
¹H-NMR ([D₆]-DMSO): δ (ppm) = 12.8 (s, breit, 1 H, COOH), 7.37 - 6.94 (m, 9 H, subst. + unsubst. Aromat), 4.89 (s, 1 H, CHOH), 3.83, 3.66 (2 H, AB-System, CH₂, ²J = 11.0 Hz), 2.80, 2.68 (2 H, AB-System, CH₂, ²J = 15.5 Hz), 1.22 (s, 3 H, 2-CH₃), 1.17 (s, 3 H, 2-CH₃). - ¹³C-NMR ([D₆]-DMSO): δ (ppm) = 173.3 (COOH), 135.6, 134.7, 134.5, 130.6 (C-quart.), 131.7, 128.1, 128.0, 127.7, 124.6 (CH, Aromaten), 123.3, 122.7, 113.5 (C-quart.), 64.5 (CHOH), 58.8 (CH₂), 42.8 (C-2), 39.4 (CH₂), 27.4 (CH₃), 27.3 (CH₃). - MS (ES⁺): m/z = 396 (27%; (M+1)⁺) Fragmente: m/z = 378 (16), 350 (100).

### Beispiel 21

### 6-(2-Benzofuranyl)-2,3-dihydro-2,2-dimethyl-1-methoxy-7-phenyl-1H-pyrrolizin-5-yl-2-oxoessigsäureethylester

### a) 6-(2-Benzofuranyl)-1-brom-2,3-dihydro-2,2-dimethyl -7-phenyl-1H-pyrrolizin-5-yl-2-oxoessigsäureethylester

6-(2-Benzofuranyl)-2,3-dihydro-2,2-dimethyl -7-phenyl-1*H*pyrrolizin-5-yl-2-oxoessigsäureethylester (4,3 g, 10 mmol, Laufer et al. Arch. Pharm. Pharm. Med. Chem. 1997, 330, 307-312.) und NBS (2,0 g, 11 mmol) werden mit AIBN (100 mg) in CCl₄ (100 mL) für 2 h am Rückfluss gekocht. Aus der abgekühlten Mischung wird das Succinimid abfiltriert und das Lösungsmittel im Vakuum entfernt und aus Diethylether kristallisiert.

Ausbeute: 4,5 g (89 %), C₂₇H₂₄BrNO₄, 506.41 g/mol
IR (KBr): 1/λ (cm⁻¹) = 2985, 2966, 1734 (CO Ester) , 1603, 1457, 1444, 1376, 1267, 1253, 1182, 1169, 1078, 1066, 1014, 760, 725, 693.
¹H-NMR ([D₆]-DMSO): δ (ppm) = 7.47-7.20 (m, 9H, 2 Ar.), 6.52 (s,1H, 3-CH Furan) ; 4.97 (s,1H,CH) ; 4,38 - 4.13 (AB , 2H,CH₂), 3.75-3.68 (m,2H,CH₂), 1,51(s,3H,CH₃), 1.28 (s,3H,CH₃) ; 1.05-0.98 (t,3H,CH₃)
¹³C-NMR ([D₆]-DMSO): δ (ppm) = 177.4, 163.7, 154.7, 148.8, 143.2, 132.5, 129.1, 128.6, 128.5, 128.4; 128.4; 127.4; 124.6; 124.1; 123.8; 123.1; 121.1; 121.1; 111.0; 107.4; 62.2; 59.1; 54.4; 48.3; 26.4; 24.9; 13.5.

### b) 6-(2-Benzofuranyl)-2,3-dihydro-2,2-dimethyl-1-methoxy-7-phenyl-1H-pyrrolizin-5-yl-2-oxoessigsäureethylester

6-(2-Benzofuranyl)-1-brom-2,3-dihydro-2,2-dimethyl-7-phenyl-1*H*pyrrolizin-5-yl-2-oxoessigsäureethylester (Beispiel 21a, 1,52 g, 3 mmol) wird in abs. MeOH (40 mL) suspendiert und 30-45 min bei Raumtemperatur bis zur Bildung eines feinen Niederschlages gerührt. Der Niederschlag wird abfiltriert und mit MeOH gewaschen.

Ausbeute: 1,17 g (86,3 %), C₂₈H₂₇NO₅ , 457.53 g/mol,
IR (KBr): 1/λ (cm⁻¹) = 1754 (CO) 1720 , 1641, 1458, 1440, 1431, 1425, 1251, 1187, 1179, 1094, 1088, 1063, 756.
¹HNMR ([D₆]-DMSO): δ (ppm) = 7.57 - 7.23 (m, 9H, 2 Ar.); 6.73 (s,1H,CH); 4.38 (s, 1H, CH); 4.15 - 4.13 (m, 2H, CH₂); 3.60 - 3.55 (s, 2H, CH₂) ; 3.02 (s, 3H, CH₃) ; 1.24 (s, 3H, CH₃) ; 1.18 (s, 3H, CH₃) ; 0.94 - 0.87 (t, 3H, CH₃)
neben wenig

### 6-(2-Benzofuranyl)-2,3-dihydro-2,2-dimethyl-1-methoxy-7-phenyl-1H-pyrrolizin-5-yl-2-oxoessigsäuremethylester

C₂₇H₂₅NO₅ , 443.50 g/mol,
¹HNMR (CDCl₃): δ (ppm) = 7.55-7.10 (m, 9H, 2 Ar.) ; 6.49 (s,1H,3-CH Furan) ; 4.21 (s, 2H, CH₂) ; 4.18 (s, 1H, CH) ; 3.35 (s, 3H, CH₃) ; 3.06 (s, 3H, CH₃) ; 1.33 (s, 3H, CH₃) ; 1.22 (s, 3H, CH₃)
¹HNMR ([D6]-DMSO) : AB-System der 3-CH₂-Gruppe

### Beispiel 22

### 6-(2-Benzofuranyl)-2,3-dihydro-2,2-dimethyl-1-methoxy-7-phenyl-1H-pyrrolizin-5-yl-2-oxoessigsäure

6-(2-Benzofuranyl)-1-brom-2,3-dihydro-2,2-dimethyl-7-phenyl-1*H*-pyrrolizin-5-yl-2-oxoessigsäureethylester (Beispiel 21a, 1,1 g, 2mmol) wird in einer methanolischen NaOH, bereitet aus Na (0,52 g, 20mmol) in MeOH (40 mL), suspendiert und 2 h am Rückfluss gekocht. Nach Entfernen des Lösemittels i. vac. wird der Rückstand in Wasser verteilt und neutrale Verbindungen mit Ethylacetat extrahiert und verworfen. Die mit HCl (10 %) auf pH 3 angesäuerte Wasserphase wird erneut mit Ethylacetat extrahiert. Die organische Phase der sauren Verbindungen wird getrocknet (Na₂SO₄) und eingeengt.

Ausbeute: 0,65 g (77 %), C₂₆H₂₃NO₅ , 429.48 g/mol,
IR (KBr): 1/λ (cm⁻¹) = 3430, 2959, 2939, 1721 (CO Säure), 1621 (sh), 1608, 1458, 1446, 1369, 1272, 1254, 1081, 1072, 1064, 752.
¹HNMR ([D6]DMSO): δ (ppm) = 7.60 - 7.15 (m, 9H, 2 Ar.) ; 6.67(s; H; CH) ; 4:34 (s, 1H, CH) ; 4.14 - 4.11 (AB, 2H, CH2) ; 3.00 (s, 3H, CH3) ; 1.24 (s, 3H, CH3) ; 1.17 (s, 3H, CH3), in CDCl₃: keine Aufspaltung der Methylengruppe an 3-Position
¹³CNMR (CDCl₃): δ (ppm) = 179,0; 165.3; 154.0; 148.6; 143.1; 133.1; 128.8; 128.4; 128.3; 127.1; 124.4; 122.9; 122.5; 122.5; 121.4; 121.2; 111.1; 107.2; 82.0; 59.7; 59.7; 57.3; 46.7; 26.1; 20.1

### Beispiel 23

### 6-(2-Benzofuranyl)-2,3-dihydro-2,2-dimethyl-1-methoxy-7-phenyl-1H-pyrrolizin-5-yl-essigsäure

6-(2-Benzofuranyl)-2,3-dihydro-2,2-dimethyl-1-methoxy-7-phenyl-1*H*-pyrrolizin-5-yl-2-oxoessigsäureethylester (Beispiel 21b, 0,92 g, 2 mmol) in Diethylenglycol (20 mL) wird mit Hydrazinhydrat 80 % (1,56 mL, 40 mmol) 30 min. auf 60 °C erwärmt, dann mit KOH techn. 85 % (2,1 g, 32 mmol) versetzt und die Mischung 4 h bei 130 °C (bis zum Ende der Gasentwicklung und Verblassen der Färbung) gehalten. Die auf Eis gegossene Mischung wird mit HCl 10 % auf pH 3 gebracht, der sich bildende feine Niederschlag wird abgesaugt und über P₂O₅ getrocknet.
Zur Reinigung wird die rohe Säure (1,0 g) an neutrales Al₂O₃ adsorbiert, Verunreinigungen durch Elution mit Diethylether entfernt, anschließend mit einer ges. NaH₂PO₄-Lösung desorbiert und in Ether aufgenommen. Der filtrierte Etherextrakt wird bis zur Trockene eingeengt.

Ausbeute: 0.45 g , C₂₆H₂₅NO₄, 415.49 g/mol.
¹HNMR ([D6]DMSO): δ (ppm) = 12,6 (b, 1H, COOH), 7.60 - 7.15 (m, 9H, 2 Ar.); 6.46 (s; 1H; 3-CH Furan), 4,23 (s, 1H, 1-CH) ; 3,90 - 3,65 (2 AB, 4H, 2 CH₂), 2.95 (s, 3H, OCH₃) ; 1.24 (s, 3H, CH₃) ; 1.13 (s, 3H, CH₃).
¹³CNMR (CDCl₃): δ (ppm) = 181,3; 163.6; 162,6, 145,4; 143.7; 138.7, 138.5, 138.1, 135.9, 133.2; 132.55, 132.43, 130,3, 128.3; 122.3; 120.5; 112.3; 92.6; 66.2; 65,7; 57,1; 41.2; 36.6; 30,45.

### Beispiel 24

### 2-[6-(2-Benzofuranyl)-2,3-dihydro-2,2-dimethyl-1-methoxy-7-phenyl-1H-pyrrolizin-5-yl]-2-hydroxy-essigsäure

Aus 2-[6-(2-Benzofuranyl)-2,3-dihydro-2,2-dimethyl-7-phenyl-1*H*-pyrrolizin-5-yl]-2-oxoessigsäureethylester (S. Laufer, et al. Arch. Pharm. Pharm. Med. Chem. 1997, 330, 307-312) nach der in Beispiel 20 beschriebenen Methode.

Ausbeute: 0,28 g (65%) aus Diethylether kristallisiert, C₂₇H₂₇NO₄, 429.52.
IR (KBr): 1/λ (cm⁻¹) = 3431, 2958,1730 (CO Ester), 1603, 1454, 1369, 1254, 1164, 1065, 1024, 751, 700.
¹HNMR (CDCl₃): δ (ppm) = 7.55 - 7.10 (m, 9H, 2 Ar.); 6.50 (d; 1H; 3-CH Furan, J=0.6Hz), 5.53 (d, 1H, CHOH, J= 2.0 Hz), 4,35 - 4,1 (ABX₃, 2H, O-CH₂CH₃); 3,88 / 3,62 (AB, 2H, J_{AB}=10.6 Hz, CH₂), 3.39 (d, OH, J=2.0 Hz), 2.80 / 2,67 (AB, 2H, CH2, J_{AB} = 15 Hz) ; 1.35 - 1.15 (2 s + t, 9H, C(CH₃)₂, OCH₂CH₃).
¹³CNMR (CDCl₃): δ (ppm) = 172.9; 154.4; 152.1, 135.9, 135.4, 129.2, 128.2, 128.1, 125.4, 123.3, 122.4, 120.3, 115.7, 114.7, 111.0, 104.1, 65.7, 62.5, 58.9, 53.5, 39.8, 27.9, 27.6, 14.1.

### Beispiel 25

### Benzoesäure-[2-(4-Chlorphenyl)-6,6-dimethyl-1-phenyl-6,7-dihydro-5H-pyrrolizin-3-yl]-ester

Eine Lösung von 6-(4-Chlorphenyl)-2,2-dimethyl-7-phenyl-2,3-dihydro-1H-pyrrolizin (1,6 g, 5 mMol, hergestellt nach Laufer et al. J. Med. Chem. 1994, 37, 1894-7), in wasserfreiem Diethylether (100 mL) wird unter Argon auf -70 °C gekühlt und bei dieser Temperatur wird eine Lösung von tert. Butyllithium (3,13 mL, 1 molar /15%ig, 5 mMol) in n-Pentan unter Rühren so zugetropft, dass die zunächst auftretende rote Färbung der Lösung wieder nach gelb umschlägt. Nach weiteren 2 h Rühren bei -70 °C lässt man die Ansatzlösung auf Raumtemperatur erwärmen und setzt das Rühren noch 1h zur Vervollständigung der Anionbildung fort, bis erneut auf -70 °C gekühlt wird. In die so vorbereitete Lösung des 3-lithiierten 2-(4-Chlorphenyl)-6,6-dimethyl-1-phenyl-6,7-dihydro-5H-pyrrolizins tropft man eine über CaCl₂ und Molekularsieb 3 Å entwässerte Lösung von Dibenzoylperoxid (1,93 g 75-80%ig angefeuchtet mit H₂O, 6 mMol) in Diethylether (50 mL) zu. Man lässt auf Raumtemperatur erwärmen und rührt bis zum vollständigen Verschwinden der Ausgangsverbindung (16 -20 h). Die Lösung wird mit Wasser versetzt und die Wasserphase mit Diethylether 2 mal (100 mL) extrahiert. Die Etherphase über Na₂SO₄ sicc. getrocknet und das Lösemittel nach Filtration vom Trokkenmittel unter vermindertem Druck vollständig entfernt. Der Rückstand wird in der Wärme aus n-Hexan zur Kristallisation gebracht. Als Kristallisat werden 1,16 g (53 % d. Th.) mit Schmelzpunkt 193 °C erhalten.

Ausbeute: 53 % = 1.16 g, C₂₈H₂₄ClNO₂, MW = 441.96
calc.: C 76.10% H 5.47% Cl 8.02% N 3.17% O 7.24%
IR (NaCl) : 1/λ (cm⁻¹) 2956; 1745; 1601; 1542; 1450; 1258; 1176; 1054; 1014; 831; 768; 699
¹HNMR (CDCl₃): δ (ppm) = 8.15-8.11 (m,2H,arom.) ; 7.70-7.40 (m,3H,arom.) ; 7.25-7.10 (m,9H,Aromat) ; 3.66 (s,2H,CH₂) ; 2.86 (s,2H,CH₂) ; 1.29 (s,6H,C(CH₃)₂);
¹³CNMR (CDCl₃): δ (ppm) = 165.0; 135.8; 134.1; 132.7; 131.2; 130.5; 130.4; 129.6; 129.1; 128.7; 128.4; 128.2; 128.1; 125.0; 113.4; 110.8; 77.2; 58.1; 43.5; 40.3; 27.8;
MS (ES⁺, 35 V), *m*/*z* = 442 / 444 ( (M+H)⁺, 100%).

### Beispiel 26

### [2-(4-Chlorphenyl)-6,6-dimethyl-1-phenyl-6,7-dihydro-5H-pyrrolizin-3-yl]-essigsäure-[2-(4-Chlorphenyl)-6,6-dimethyl-1-phenyl-6,7-dihydro-5H-pyrrolizin-3-yl]-ester

Eine Lösung von 2-(4-Chlorphenyl)-6,6-dimethyl-1-phenyl-6,7-dihydro-5H-pyrrolizin-3-yl]-essigsäure (38,0 g; 0,1 Mol) in wasser freiem Diethylether (400 mL) wird mit BF₃-Etherat (2 mL, 2.26 g, 0.016 Mol) bei Raumtemperatur versetzt und 30 min. gerührt. Die in dieser Zeit violett verfärbte Lösung wird anschließend unter vermindertem Druck eingeengt (200 mL) und mit n-Heptan (200 mL) aufgefüllt. Bei vermindertem Druck wird weiter Ether (150 mL) abdestilliert und der kristallisierende, violettgefärbte Rückstand von der Ether-Heptan-Mutterlauge getrennt. Die Mutterlauge wird bis zur Trockne konzentriert (5.5 g) und in Ethanol (50 mL) in der Wärme aufgenommen, nach dem Kaltstellen (16 h) wird die blaue Lösung vom unlöslichen Rückstand abgesaugt. Das Kristallisat wird mit eiskaltem Ethanol nachgewaschen, bei Bedarf aus wenig Ethanol (10 mL) nochmals umkristallisiert. Es werden 0,45 g der gesuchten Verbindung erhalten. Die Substanz kann auch säulenchromatographisch (SiO₂, Hexan Ether 9:1 ) gereinigt (rf 0.2, Ethylester rf 0.22) werden.

Ausbeute: 1,3 % = 0.45 g, C₄₄H₄₀Cl₂N₂O₂, MW = 699.73
calc.: C 75.53% H 5.76% Cl 10.13% N 4.00% O 4.57%
gef.: C 75.14% H 5.75% N 4.07%
IR (NaCl): 1/λ (cm⁻¹) = 2960; 2872; 1768; 1601; 1541; 1536; 1451; 1112; 1094; 1012; 833; 698 -
¹HNMR (CDCl₃): δ (ppm) = 7.25-7.01 (m,18,arom.) ; 3.71 (s,2H,CH₂) ; 3.52 (s,2H,CH₂) ; 3.41 (s,2H,CH₂) ; 2.81 (s,2H,CH₂) ; 2.78 (s,2H,CH₂) ; 1.27 (s,6H,C(CH₃)₂) ; 1.15 (s,6H,C(CH₃)₂)
¹³CNMR (CDCl₃): δ (ppm) = 27.80; 31.06; 40.17; 40.37; 43.06; 43.51; 57.81; 58.21; 110.66; 113.33; 115.06; 115.91; 124.14; 124.77; 125.07; 128.09; 128.12; 128.13; 128.24; 128.35; 128.44; 129.04; 129.36; 130.41; 131.19; 131.56; 131.84; 132.47; 134.58; 134.66; 135.50; 135.74; 169.13
MS (70 eV, PI-FDMS, +VE, +HMR, B-Scan), *m*/*z* = 698.2 (M⁺, 100%) 699.2 (M⁺, 50%), 700.2 (M⁺,75%), 701.2 (M⁺, 35%), 702.3 (M⁺, 18%),. MS (ES⁺, 35 V), *m*/*z* (% rel. Intens.) (M+H)⁺ : 699 (32), 700 (18), 701 (20), 702 (10), 703 (7); 334 (65), 335 (15), 336 (100), 337 (25), 338 (80), 339 (18), 340 (19).
MS (EI, 70 eV), *m*/*z* (% rel. Intens.) (M⁺ : 698 (1), 699,(0,7), 700 (1), 334 (100), 335 (42), 336 (60), 337 (58), 338 (18), 339 (15).

### Beispiel 27

### 2-(4-Chlorphenyl)-7a-hydroxy-6,6-dimethyl -1-phenyl-5,6,7,7a-tetrahydro-pyrrolizin-3-on

Der in Beispiel 25 beschriebene Benzoesäure-[2-(4-Chlorphenyl)-6,6-dimethyl-1-phenyl-6,7-dihydro-5H-pyrrolizin-3-yl]-ester (0.4 g, 0.9 mMol) wird in MeOH (20 mL) suspendiert und mit NaOH-Lösung (1 mL, 10%ig) versetzt und anschließend 3 h bis zum Verschwinden des Eduktes (dc: SiO₂/Diisopropylether, rf 0.95) unter Rückfluss gekocht, anschließend wird der Alkohol im Vakuum entfernt. Der wässrige Rückstand wird mit Wasser (20 mL) und Diethylether (30 mL) versetzt und mit HCl (10%ig) sauer gestellt (pH 2-3). Die Etherphase wird abgetrennt, die Wasserphase dreimal mit Ether (100 mL) extrahiert und die gesammelten Etherphasen vereint, über Na₂SO₄ sicc. getrocknet und das Lösemittel unter vermindertem Druck eingeengt. Als Rückstand verbleiben 0.39 g Feststoff eines Substanzgemisches, das sc (SiO₂/Diisopropylether, rf 0.55) gereinigt wird. Fraktionen 8-13 (zu je 10 mL) enthalten 120 mg (40%) der Titel-Verbindung.

Ausbeute: 40 % = 120 mg, C₂₁H₂₀ClNO₂, MW = 353.85
calc.: C 71.28% H 5.70% Cl 10.02% N 3.96% O 9.04%
IR (NaCl): 1/λ (cm⁻¹) = 3355; 1680; 1394; 1092; 1068; 696; 530;
¹HNMR (DMSO-d₆): δ (ppm) = 7.45- 7.23 (m,9H,Aromat) ; 3.60 / 3.22 (AB, CH₂, J_{AB}=11,5 Hz) ; 2.195 / 1.962 (AB,CH₂, J_{AB}=11,5 Hz); 1.367 (s,3H,CH₃) ; 1.067 (s,3H,CH₃)
MS (ES⁺, 35 V), *m*/*z* (% rel. Intens.) MH⁺ : 354 (100), 355 (20), 356 (35), 357 (7); (MH-H₂O)⁺: 336 (40), 337 (5), 338 (10), 339 (2).

Das als Verseifungsprodukt erwartete 2-(4-Chlorphenyl) -6,6-dimethyl -1-phenyl-5,6,7,7a-tetrahydro-pyrrolizin-3-on (Keto-Tautomer des 2-(4-Chlorphenyl)-6,6-dimethyl-1-phenyl-6,7-dihydro-5H-pyrrolizin-3-ol) konnte in keinem Fall beobachtet werden.
Nach längerem Stehen (16 h) einer CHCl₃-Lösung des Produktes wird das in Beispiel 29 beschriebene 2-(4-Chlorphenyl)-6,6-dimethyl-1-phenyl-5,6-dihydro-pyrrolizin-3-on als Substanz in reiner Form erhalten (H₂O-Elimination).

### Beispiel 28

### 2-(4-Chlorphenyl)-6,6-dimethyl-1-phenyl-6,7-dihydro-pyrrolizin-3-on (Acetoxylierungsmethode nach L. Eberson und L. Jönsson, Acta chem. Scand. Ser. B 30, 361 (1976)

6-(4-Chlorphenyl)-2,2-dimethyl-7-phenyl-2,3-dihydro-1H-pyrrolizin (1,6 g, 5 mMol, s.o.), Dikaliumperoxodisulfat (1,62 g, 6 mMol), Palladium-(II)-acetat (0.05 g, 0,2 mMol) und 2,2'-Bipyridyl (0,02 g, 0.1 mMol) werden in wasserfreier Essigsäure (20 mL) suspendiert und 4 h bei 100 °C gerührt (Anm.: Zuvor war bei Raumtemperatur nach 4 h keine Umsetzung zu beobachten.) .
Die schwarze Eisessig-Lösung wird mit Na₂CO₃-Lösung abgestumpft (pH 7) und dreimal mit Ether extrahiert (150 mL). Die Etherlösung wird mit NaHCO₃-Lösung gewaschen, über Na₂SO₄ sicc. getrocknet und das Lösemittel wird unter vermindertem Druck entfernt. Der Rückstand wird über eine kurze Säule von SiO₂ mit n-Hexan/Diisopropylether als Eluens gereinigt.

Ausbeute: 88 % = 1.48 g, C₂₁H₁₈ClNO, MW = 335.84
calc.: C 75.11% H 5.40% Cl 10.56% N 4.17% O 4.76%
IR (NaCl): 1/λ (cm⁻¹) = 2956; 1689; 1483; 1379; 1093; 833; 739; 696
¹HNMR (CDCl₃): δ (ppm) = 7.43- 7.24 ( m,9H,Aromat ) ; 5.61 (s,1H,CH) ; 3.68 (s,2H,CH₂) ; 1.35 (s,6H,C(CH₃)₂)
MS (ES⁺, 35 V), *m*/*z* (% rel. Intens.) MH⁺ : 336 (100), 337 (15), 338 (37), 339 (7).

Dieselbe Substanz bildet sich beim Stehen in Lösung (zBsp CHCl₃) aus der Substanz von Beispiel Nr. 28 (s.o.).

## Patentansprüche

1. Pyrrolizinverbindungen der Formel I 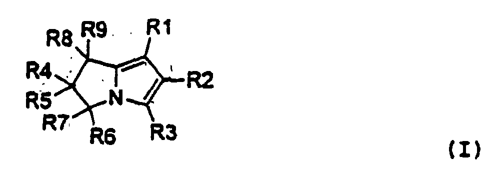 worin
R1 und R2, die gleich oder verschieden sein können, für Aryl oder einen aromatischen mono- oder bicyclischen, heterocyclischen Rest stehen, der 1, 2 oder 3 Heteroatome aufweist, die unabhängig voneinander ausgewählt sind unter N, O und S, wobei die Reste R1 und R2 gegebenenfalls durch 1, 2 oder 3 Gruppen, die unabhängig voneinander ausgewählt sind unter Alkyl, Halogen CF₃, Hydroxy, Alkoxy, Aryloxy und CN, substituiert und gegebenenfalls mit Phenyl oder Naphthyl kondensiert sein können;
R3 für H, Alkyl, COOH, COOAlkyl, COOAlkPhenyl, COCOOH, COCOOAlkyl, CHO oder A-Y steht, wobei
A für C₁₋C₈-Alkylen oder C₂-C₈-Alkenylen steht, das gegebenenfalls durch Hydroxyl oder Alkoxy substituiert sein kann,
Y für COOH, SO₃H, OPO(OH)₂, OP(OH)₂, Tetrazolyl, COO-Alkyl, SO₃Alkyl, CHO oder OH steht;
Alk für C₁-C₄-Alkylen steht;
R4, R5, R6 und R7, die gleich oder verschiedenen sein können, für H, Alkyl, Hydroxylalkyl oder Alkoxyalkyl stehen;
einer der Reste R8 und R9 für H, Alkyl, Hydroxyalkyl oder Alkoxyalkyl steht und der andere für Hydroxyl, Alkoxy oder Acyloxy steht oder R8 und R9 zusammen mit dem Kohlenstoffatom, an das sie gebunden sind, für eine Carbonylgruppe stehen,
und die optischen Isomere, physiologisch verträglichen Salze und physiologisch leicht hydrolysierbaren Ester davon.

2. Verbindungen der Formel I nach Anspruch 1, wobei R3 für H, Alkyl, COOH, CHO oder A-Y, wobei A für C₁-C₈-Alkylen, das gegebenenfalls durch OH substituiert ist, und Y für COOH, SO₃H, OPO(OH)₂, CHO oder Tetrazolyl stehen.

3. Verbindungen der Formel I nach Anspruch 1 oder 2, wobei R3 für H, Alkyl, COOH, CHO oder A-Y steht, wobei A für C₁-C₈-Alkylen, das gegebenenfalls durch OH substituiert ist, und Y für COOH stehen.

4. Verbindungen der Formel I nach einem der vorhergehenden Ansprüche, wobei R1 und R2, die gleich oder verschieden sein können, für Aryl oder einen 5- oder 6-gliedrigen heterocyclischen, aromatischen Rest stehen, wobei R1 und R2 unabhängig voneinander 1 oder 2 Halogen-, Hydroxy-, Alkoxy- und/oder CF₃-Substituenten aufweisen können und wobei der heterocyclische Rest mit einer Phenylgruppe kondensiert sein kann.

5. Verbindungen der Formel I nach Anspruch 4, wobei R1 und R2, die gleich oder verschieden sein können, für Phenyl, mit 1 oder 2 Halogenatomen oder Hydroxygruppen substituiertes Phenyl, Thienyl oder Benzofuranyl stehen.

6. Verbindungen der Formel I nach Anspruch 4, wobei R1 für Phenyl und R2 für monohalogen- oder monohydroxy-substituiertes Phenyl oder Benzofuranyl stehen.

7. Verbindungen der Formel I nach einem der vorhergehenden Ansprüche, wobei R4 für Hydroxyalkyl, R5 für H oder Alkyl und R6 und R7 unabhängig voneinander für H oder Alkyl stehen.

8. Verbindungen der Formel I nach einem der vorhergehenden Ansprüche, wobei R8 und R9 zusammen mit dem Kohlenstoffatom, an das sie gebunden sind, für eine Carbonylgruppe stehen.

9. Pharmazeutisches Mittel, enthaltend wenigstens eine Verbindung nach einem der Ansprüche 1 bis 8, gegebenenfalls zusammen mit pharmazeutisch akzeptablen Träger- und/oder Zusatzstoffen.

10. Verwendung wenigstens einer Verbindung nach einem der Ansprüche 1 bis 8 zur Herstellung eines pharmazeutischen Mittels zur Prävention von allergisch induzierten Erkrankungen oder zur Behandlung von Erkrankungen des rheumatischen Formenkreises.

## Claims

1. A pyrrolizine compound of the formula I 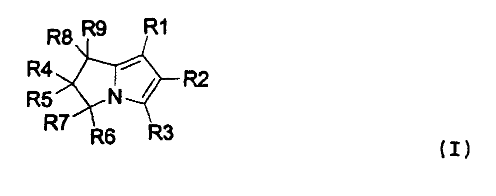 in which
R1 and R2, which can be identical or different, are aryl or an aromatic mono- or bicyclic, heterocyclic radical which has 1, 2 or 3 heteroatoms which independently of one another are selected from N, O and S, where the radicals R1 and R2 can optionally be substituted by 1, 2 or 3 groups, which independently of one another are selected from alkyl, halogen, CF₃, hydroxy, alkoxy, aryloxy and CN, and can optionally be fused to phenyl or naphthyl;
R3 is H, alkyl, COOH, COOalkyl, COOalkphenyl, COCOOH, COCOOalkyl, CHO or A-Y, where
A is C₁-C₈-alkylene or C₂-C₈-alkenylene, which can optionally be substituted by hydroxyl or alkoxy,
Y is COOH, SO₃H, OPO(OH)₂, OP(OH)₂, tetrazolyl, COOalkyl, SO₃alkyl, CHO or OH;
Alk is C₁-C₄-alkylene;
R4, R5, R6 and R7, which can be identical or different, are H, alkyl, hydroxylalkyl or alkoxyalkyl;
one of the radicals R8 and R9 is H, alkyl, hydroxyalkyl or alkoxyalkyl and the other is hydroxyl, alkoxy or acyloxy or R8 and R9, together with the carbon atom to which they are bonded, are a carbonyl group,
and the optical isomers, physiologically tolerable salts and physiologically readily hydrolyzable esters thereof.

2. A compound of the formula I as claimed in claim 1, where R3 is H, alkyl, COOH, CHO or A-Y, where A is C₁-C₈-alkylene which is optionally substituted by OH, and Y is COOH, SO,H, OPO(OH)₂, CHO or tetrazolyl.

3. A compound of the formula I as claimed in claim 1 or 2, where R3 is H, alkyl, COOH, CHO or A-Y, where A is C₁-C₈-alkylene which is optionally substituted by OH, and Y is COOH.

4. A compound of the formula I as claimed in one of the preceding claims, where R1 and R2, which can be identical or different, are aryl or a 5- or 6-membered heterocyclic, aromatic radical, where R1 and R2 independently of one another can contain 1 or 2 halogen, hydroxy, alkoxy and/or CF₃ substituents and where the heterocyclic radical can be fused to a phenyl group.

5. A compound of the formula I as claimed in claim 4, where R1 and R2, which can be identical or different, are phenyl, phenyl which is substituted by 1 or 2 halogen atoms or hydroxy groups, thienyl or benzofuranyl.

6. A compound of the formula I as claimed in claim 4, where R1 is phenyl and R2 is monohalo- or monohydroxy-substituted phenyl or benzofuranyl.

7. A compound of the formula I as claimed in one of the preceding claims, where R4 is hydroxyalkyl, R5 is H or alkyl and R6 and R7 independently of one another are H or alkyl.

8. A compound of the formula I as claimed in one of the preceding claims, where R8 and R9, together with the carbon atom to which they are bonded, are a carbonyl group.

9. A pharmaceutical composition, comprising at least one compound as claimed in one of the claims 1 to 8, if appropriate together with pharmaceutically acceptable vehicles and/or additives.

10. The use of at least one compound as claimed in one of the claims 1 to 8 for the production of a pharmaceutical composition for the prevention of allergically induced disorders or for the treatment of disorders of the rheumatic type.

## Revendications

1. Composés de pyrrolizine répondant à la formule I : 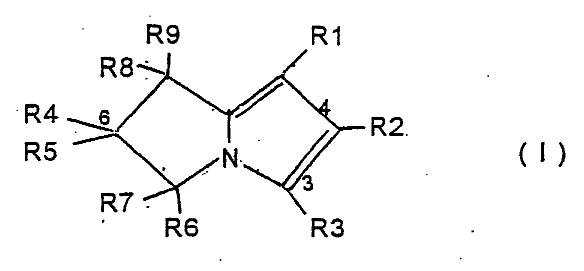 dans laquelle :
R1 et R2, qui peuvent être identiques ou différents, désignent un radical aryle ou un radical aromatique hétérocyclique mono ou bicyclique hétérocyclique comportant 1, 2, ou 3 hétéroatomes, qui peuvent être choisis indépendamment l'un de l'autre parmi N, O et S, les radicaux R1 et R2 pouvant aussi être substitués par 1, 2, ou 3 groupes choisis indépendamment parmi les groupes alkyle, halogène, CF₃, hydroxy, alkoxy, aryloxy et CN, ou être condensés avec un groupe phényle ou naphtyle,
R3 désigne H ou un groupe alkyle, COOH, COO-alkyle, COO-*alk*-phényle, COCOOH, COCOO-alkyle, CHO ou un groupe A-Y où
A est un radical alkyle en C₁-C₈ ou alcényle en C₂-C₈ qui peut être éventuellement substitué par un radical hydroxyle ou alkoxy,
Y est COOH, SO₃H, OPO(OH)₂, OP(OH)₂, tétrazolyle, COO-alkyle, SO₃-alkyle, CHO ou OH,
*alk* est un groupe alkyle en C₁-C₄,
R4, R5, R6 et R7, qui peuvent être identiques ou différents, sont H ou un groupe alkyle, hydroxyalkyle ou alkoxyalkyle,
l'un des radicaux R8 et R9 désigne H ou un groupe alkyle, hydroxyalkyle ou alkoxyalkyle et l'autre un groupe hydroxyle, alkoxy ou acyloxy, ou les deux radicaux R8 et R9 forment ensemble un groupe carbonyle avec un atome de carbone auquel ils sont liés,
ainsi que leurs isomères optiques, leurs sels physiologiquement compatibles et leurs esters à hydrolyse physiologique facile.

2. Composés suivant la formule I de la revendication 1 dans laquelle R3 est H, un groupe alkyle, COOH, CHO ou A-Y où A est un groupe alkyle en C₁-C₈, éventuellement substitué OH, et Y est COOH, SO₃H, OPO(OH)₂, OP(OH)₂, CHO, ou un groupe tétrazolyle.

3. Composés suivant la formule I de la revendication 1 ou 2, dans lesquels R3 est H, un groupe alkyle, COOH, CHO ou A-Y où A est un groupe alkyle en C₁-C₈, éventuellement substitué OH, et Y est COOH.

4. Composés présentant la formule I suivant l'une quelconque des revendication précédentes dans lesquels R1 et R2 peuvent être identiques ou différents et désignent chacun un groupe aryle ou un groupe aromatique hétérocyclique à 5 ou 6 chaînons, où R1 et R2 peuvent indépendamment l'un de l'autre présenter un ou deux substituants halogène, hydroxy, alkoxy et/ou CF₃, et où le radical hétérocyclique peut être condensé en un groupe phényle.

5. Composés présentant la formule I suivant la revendication 4 dans lesquels R1 et R2 peuvent être identiques ou différents et désignent chacun un groupe phényle, un groupe phényle substitué par un ou deux atomes d'halogène ou groupes hydroxy, un groupe thiényle ou un groupe benzofuranyle.

6. Composés présentant la formule I suivant la revendication 4 dans lesquels R1 est un groupe phényle et R2 est un groupe phényle mono-substitué par un atome d'halogène ou un groupe hydroxy ou un groupe benzofuranyle.

7. Composés présentant la formule 1 suivant l'une quelconque des revendications précédentes dans lesquels R4 est un groupe hydroxyalkyle, R5 est H ou un radical alkyle et R6 et R7 désignent indépendamment l'un de l'autre un atome d'hydrogène ou un groupe alkyle.

8. Composés présentant la formule I suivant l'une quelconque des revendications précédentes dans lesquels R8 et R9 forment ensemble, avec un atome de carbone auquel ils sont liés, un groupe carbonyle.

9. Produits pharmaceutiques, comportant au moins un composé suivant l'une quelconque des revendications 1 à 8, éventuellement complété par un véhicule et/ou des additifs pharmaceutiquement compatibles.

10. Utilisation de l'un au moins des composés suivant l'une quelconque des revendications 1 à 8 pour la préparation d'un produit pharmaceutique pour la prévention de maladies induites par allergie et pour le traitement de maladies á forme rhumatismale.
